(19)

**Europäisches Patentamt**

**European Patent Office**

**Office européen des brevets**

(11) **EP 4 296 354 A1**

(12) **EUROPEAN PATENT APPLICATION**

(43) Date of publication:
**27.12.2023 Bulletin 2023/52**

(21) Application number: **22180986.6**

(22) Date of filing: **24.06.2022**

(51) International Patent Classification (IPC):
**C12N 1/20** $^{(2006.01)}$    **C12P 13/04** $^{(2006.01)}$

(52) Cooperative Patent Classification (CPC):
**C12P 13/04; C12N 1/20;** C12R 2001/01

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Arkeon GmbH**
**3430 Tulln an der Donau (AT)**

(72) Inventors:
• **RITTMANN, Simon Karl-Maria Rasso**
**3430 Tulln an der Donau (AT)**

• **SCHUPP, Benjamin**
**3430 Tulln an der Donau (AT)**
• **REISCHL, Barbara**
**3430 Tulln an der Donau (AT)**
• **TAUBNER, Ruth-Sophie**
**3430 Tulln an der Donau (AT)**
• **PALABIKYAN, Hayk**
**3430 Tulln an der Donau (AT)**
• **FINK, Christian**
**3430 Tulln an der Donau (AT)**

(74) Representative: **SONN Patentanwälte GmbH & Co KG**
**Riemergasse 14**
**1010 Wien (AT)**

(54) **METHOD FOR PRODUCING AMINO ACIDS IN A BIOREACTOR WITH METHANOGENIC MICROORGANISMS**

(57)    The present invention provides a method of producing amino acids by fermentation in a bioreactor, wherein the bioreactor comprises methanogenic microorganisms in a fermentation broth, the method comprising at least the step of feeding a gaseous carbon source comprising carbon dioxide and/or carbon monoxide, a nitrogen source comprising nitrogen gas and preferably a sulfur source to the bioreactor under conditions such that the methanogenic microorganisms produce the amino acids, wherein the fermentation broth comprises ammonium at a concentration of at least 0.1 mmol/L and the tungstate concentration in the fermentation broth is below 0.1 pmol/L. Preferred methanogenic microorganisms are e.g. archaea selected from *Methanothermobacter, Methanothermococcus* and *Methanococcus.* Also provided is a fermentation broth comprising methanogenic microorganisms, ammonium at a concentration of at least 0.1 mmol/L, wherein the tungstate concentration in the fermentation broth is below 0.1 pmol/L, and a bioreactor comprising the same.

EP 4 296 354 A1

**Description**

**TECHNICAL FIELD**

The field of present invention relates to methods for producing amino acids by fermentation in a bioreactor with methanogenic microorganisms.

**BACKGROUND**

Amino acids are applied in a variety of sectors: food and feed, agriculture, pharmaceuticals and even packaging and housing. The biosynthesis of proteinogenic amino acids is an important branch of biotechnology (Becker and Wittmann 2012). However, the metabolic potential of archaea with regard to amino acid production has up to now been vastly overlooked (Pfeifer et al. 2021).

Methanogenic archaea are known for the ability to generate methane ($CH_4$) as end product of their energy metabolism (see e.g. Mand & Metcalf, 2019). Some of them are able to grow autotrophic, hydrogenotrophic by reducing carbon dioxide ($CO_2$) with molecular hydrogen ($H_2$) to $CH_4$, and play a crucial role in the global carbon cycle (Lyu et al. 2018). According to their substrate utilization spectrum, methanogens can be divided into different metabolic groups: hydrogenotrophic ($H_2$, formate or simple alcohols), aceticlastic (acetate), methylotrophic (compounds containing a methyl group), $H_2$ dependent methylotrophic (methylated compounds with $H_2$ as electron donor), and methoxydotrophic (methoxylated aromatic compounds) (Mayumi et al. 2016, Kurth et al. 2020).

Taubner et al. is an exobiology research paper unrelated to biotechnology. Specifically, the document concerns the membrane lipid composition and amino acid excretion patterns of *Methanothermococcus okinawensis* grown in the presence of inhibitors detected in the Enceladian plume. These findings are important for understanding the eco-physiology of methanogens on Earth and have implications for the use of biomarkers as possible signs of extraterrestrial life for future space missions in the Solar System. Along similar lines, Taubner et al, 2018, discusses biological methane production under putative Enceladus-like conditions.

Rittmann et al, 2021, generally concerns the use of archaea in biotechnology.

Several studies discuss the use of methanogenic archaea in renewable energy production by reducing $CO_2$ with $H_2$ to $CH_4$ (Pappenreiter et al. 2019, Rittmann et al. 2018, Mauerhofer et al. 2018, Abdel Azim et al. 2018, Abdel Azim et al. 2017, Rittmann 2015, Mauerhofer et al. 2021, Rittmann et al, 2012). Liu et al., 2021, concerns the effects of different amino acids and their configurations on methane yield and biotransformation of intermediate metabolites during anaerobic digestion. WO 2012/110256 A1 discloses a method of converting carbon dioxide and hydrogen to methane by methanogenic microorganisms. WO 2014/128300 A1 relates to a method and system for producing methane using methanogenic microorganisms and applying specific nitrogen concentrations in the liquid phase.

WO 2017/070726 A1 relates to a method for determining the culture status of microbe cultures, such as cultures with hydrogenotrophic methanogenic microorganisms.

WO 2016/179545 A1 discloses compositions and methods for the biological production of methionine.

US 2019/0194630 A1 relates to compositions and methods for biological production of amino acids in hydrogenotrophic microorganisms. In particular, the hydrogenotrophic microorganism may be chosen from *Methanococcus* and *Methanosarcina.*

WO 2020/252335 A1 relates to processes and systems for producing products by fermentation. Disclosed is in particular a process comprising (a) feeding a gaseous mixture comprising $CO_x$ and $H_2$, where x is 1 or 2, a nitrogen source, and optionally a sulfur source to a bioreactor that contains hydrogenotrophic microorganisms under conditions such that the hydrogenotrophic microorganisms produce at least one fermentation product chosen from an amino acid, an alcohol, aldehyde or ketone, a carboxylic acid, or a hydroxyl or keto acid; (b) removing a gas stream from the bioreactor, the gas stream having at least one compound chosen from a sulfur containing compound, a nitrogen containing compound, $H_2$, $CO_x$, and a hydrocarbon compound, where x is 1 or 2; (c) removing a liquid stream from the bioreactor comprising fermentation broth, hydrogenotrophic microorganisms, and fermentation product; and (d) separating the hydrogenotrophic microorganisms from the liquid stream and recycling the hydrogenotrophic microorganisms back to the bioreactor. The hydrogenotrophic microorganisms may be chosen from methanogenic archaea.

Despite these efforts, there is still a need for further development of fermentative methods with methanogenic microorganisms.

It is thus an object of the present invention to provide improved methods for producing amino acids by fermentation in a bioreactor with methanogenic microorganisms. These methods should make more efficient use of (natural) resources, be more environmentally friendly, lead to the reduction of greenhouse gas emissions, have an increased yield and/or overcome one or more disadvantages of amino acid production methods known in the art.

## SUMMARY OF THE INVENTION

The present invention provides a method for producing amino acids by fermentation in a bioreactor. The bioreactor comprises methanogenic microorganisms in a fermentation broth. This method comprises at least the step of feeding a gaseous carbon source comprising carbon dioxide and/or carbon monoxide, a nitrogen source comprising nitrogen gas (i.e. $N_2$ gas or molecular nitrogen gas) and preferably a sulfur source to the bioreactor under conditions such that the methanogenic microorganisms produce the amino acids. The fermentation broth comprises ammonium (i.e. $NH_4^+$) at a concentration of at least 0.1 mmol/L, preferably at least 1.0 mmol/L, more preferably at least 2.0 mmol/L or even at least 4.0 mmol/L (e.g. 0.1 mmol/L to 200 mmol/L, preferably 2 mmol/L to 100 mmol/L, more preferably 4 to 40 mmol/L). The tungstate concentration (in particular the orthotungstate, i.e. $WO_4^{-2}$, concentration) in the fermentation broth is below 0.1 $\mu$mol/L, preferably below 0.01 $\mu$mol/L, especially below 0.001 $\mu$mol/L (in particular, the fermentation broth is essentially free of tungstate). Typically, this method comprises the step of harvesting at least a portion of the amino acids from the bioreactor (which is e.g. followed by purification methods to separate the amino acids from other constituents of the fermentation broth).

In an aspect, the present invention provides the use of methanogenic microorganisms (e.g. any of the methanogenic archaea disclosed herein, such as for instance *Methanothermobacter, Methanothermococcus, Methanocaldococcus* and *Methanococcus*) for producing amino acids from an electron donor compound, a gaseous carbon source comprising carbon dioxide and/or carbon monoxide, a nitrogen source comprising nitrogen gas and preferably a sulfur source in a fermentation broth. Also in this aspect, the fermentation broth comprises ammonium at a concentration of at least 0.1 mmol/L, preferably at least 1.0 mmol/L, more preferably at least 2.0 mmol/L or even at least 4.0 mmol/L (e.g. 0.1 mmol/L to 200 mmol/L, preferably 2 mmol/L to 100 mmol/L, more preferably 4 to 40 mmol/L). The tungstate concentration (in particular the orthotungstate, i.e. $WO_4^{-2}$, concentration) in the fermentation broth is below 0.1 $\mu$mol/L, preferably below 0.01 $\mu$mol/L, especially below 0.001 $\mu$mol/L (in particular, the fermentation broth is essentially free of tungstate). It is preferred that the electron donor compound is selected from hydrogen gas (i.e. $H_2$ or molecular hydrogen gas), acetate, a methyl compound preferably selected from methylamines, methyl sulfides and methanol, any other alcohol, preferably a secondary alcohol such as 2-propanol or 2-butanol, a methoxylated aromatic compound, formate, and mixtures thereof. Hydrogen gas, methanol and/or acetate are particularly preferred as electron donor compounds.

In another aspect, the present invention provides a fermentation broth comprising methanogenic microorganisms (e.g. any of the methanogenic archaea disclosed herein, such as for instance *Methanothermobacter, Methanothermococcus, Methanocaldococcus* and *Methanococcus*). Also in this aspect, the fermentation broth comprises ammonium at a concentration of at least 0.1 mmol/L, preferably at least 1.0 mmol/L, more preferably at least 2.0 mmol/L or even at least 4.0 mmol/L (e.g. 0.1 mmol/L to 200 mmol/L, preferably 2 mmol/L to 100 mmol/L, more preferably 4 to 40 mmol/L). The tungstate concentration (in particular the orthotungstate, i.e. $WO_4^{-2}$, concentration) in the fermentation broth is below 0.1 $\mu$mol/L, preferably below 0.01 $\mu$mol/L, especially below 0.001 $\mu$mol/L (in particular, the fermentation broth is essentially free of tungstate).

In yet another aspect, the present invention provides a bioreactor comprising this fermentation broth.

Agriculture and the production of artificial nitrogen-containing fertilizers are an indirect source of greenhouse gas emissions through releasing $N_2O$ via nitrification of ammonia ($NH_3$). The Haber-Bosch processes is the main industrial procedure for synthetic $N_2$ fixation and responsible for a release of 1.5 tons of $CO_2$ per ton of $NH_3$ produced. In the course of the present invention, the inventors examined whether a biological process could be used for combined carbon and $N_2$ fixation and concomitant amino acid production. Surprisingly, it turned out that $NH_4^+$ addition to the fermentation medium was advantageous for enabling efficient $N_2$ fixation in methanogenic microorganisms. In addition, it turned out that it was highly beneficial that the medium contained as little tungstate as possible (i.e. only below 0.1 $\mu$mol/L, preferably below 0.01 $\mu$mol/L, especially below 0.001 $\mu$mol/L), as amino acid yield under nitrogen fixing conditions was lower otherwise. Accordingly, in the course of the present invention, the inventors found $NH_4^+$ and tungstate concentrations most suitable for efficient amino acid production (and active secretion into the cell culture supernatant) in methanogenic microorganisms during combined carbon and $N_2$ fixation.

## DETAILED DESCRIPTION OF THE INVENTION

The detailed description given below relates to all of the above aspects of the invention unless explicitly excluded.

Biological molecular nitrogen ($N_2$) fixation is a key process in the global nitrogen cycle where it is closely linked to the carbon cycle. With 16 moles ATP per mol $N_2$ fixed, it is one of the most expensive metabolic processes (Thamdrup 2012; Hu and Ribbe 2012). Certain phyla of archaea, but also bacteria, are able to fix $N_2$ (Fernandez et al. 2019). This biological process is referred to as diazotrophy. Diazotrophic growth was first reported among the archaea in 1984, when *Methanosarcina barkeri* (Murray and Zinder 1984) and *Methanococcus thermolithotrophicus* (Belay et al. 1984) were shown to be able to fix $N_2$. The enzymes needed for the reduction of $N_2$ are nitrogenases, which are encoded in the *nif* gene cluster (Raymond et al. 2004).

When the nitrogen source comprises nitrogen gas, a certain ammonium concentration range has turned out to be especially advantageous for energy production (under $N_2$-fixing conditions). It is thus preferred that the ammonium concentration in the fermentation broth is at least 0.1 mmol/L, preferably at least 1.0 mmol/L, more preferably at least 2.0 mmol/L or even at least 4.0 mmol/L; or 0.1 mmol/L to 200 mmol/L, preferably 2 mmol/L to 100 mmol/L, more preferably 4 mmol/L to 40 mmol/L, even more preferably 5 mmol/L to 35 mmol/L, yet even more preferably 6 mmol/L to 30 mmol/L, especially 7 mmol/L to 25 mmol/L, or even 10 mmol/L to 20 mmol/L. It is evident to the skilled person that, especially in continuous culture, the concentration in the broth may be subject to fluctuations (until a steady state is reached). It is however preferred that the ammonium concentration stays above at least 0.1 mmol/L, preferably at least 1.0 mmol/L, more preferably at least 2.0 mmol/L or even at least 4.0 mmol/L or within any of the aforementioned ranges (e.g. 0.1 mmol/L to 200 mmol/L or 10 mmol/L to 20 mmol/L) for at least 5 min, preferably at least 10 min, even more preferably at least at least 20 min, yet even more preferably at least 1 h, especially at least 5 h or even at least 10 h (or at least 20 h or at least 40 h).

In a preferred embodiment, an electron donor (or electron donor compound) suitable for the methanogenic microorganisms is fed to the bioreactor. In particular, hydrogen gas (i.e. molecular hydrogen or $H_2$), acetate, a methyl compound preferably selected from methylamines, methyl sulfides and methanol, any other alcohol, preferably a secondary alcohol such as 2-propanol or 2-butanol, a methoxylated aromatic compound and/or formate are fed to the bioreactor (as electron donor compounds). Hydrogen gas, acetate, methanol or combinations thereof (e.g. methanol and acetate) are particularly preferred.

According to a further preferred embodiment, methane (produced by the methanogenic microorganisms) is harvested from the bioreactor.

Typically, the inventive method comprises harvesting at least a portion of the amino acids from the bioreactor. Especially continuous culture, this may involve removing a liquid stream from the bioreactor, the liquid stream comprising fermentation broth, methanogenic microorganisms, and produced amino acids (in particular in the culture supernatant), separating the methanogenic microorganisms from the liquid stream (e.g. by filtration) and recycling the methanogenic microorganisms back to the bioreactor. The harvested amino acids (which may be present in a liquid fraction of the fermentation broth) are then preferably further purified, e.g. by methods known in the art such as chromatography.

Surprisingly, it turned out that methanogenic microorganisms actively excrete (or secrete) many different amino acids into the culture supernatant. The secretion of these amino acids to the supernatant is especially remarkable, as it simplifies downstream steps (e.g. no cell lysis required for harvesting the product) while at the same time increasing productivity (as methanogenic microorganisms remain viable and can remain or be fed back into the bioreactor).

Therefore, according to a preferred embodiment of the present invention, the method comprises harvesting at least a portion of the amino acids from the supernatant of the fermentation broth.

In the course of the present invention, it turned out that many of the methanogenic microorganisms remained viable and/or intact (as lysis was much reduced). It is thus preferred that the supernatant (used in the harvesting step) has a protein content of less than 1000 μg/mL, preferably less than 500 μg/mL, more preferably less than 250 μg/mL, even more preferably less than 100 μg/mL, yet even more preferably less than 50 μg/ml or even less than 40 μg/mL, especially less than 30 μg/mL or even less than 20 μg/mL. The protein content may be measured by methods known in the art, e.g. Bradford protein assay.

Along similar lines, is highly preferred that at least 50%, preferably at least 60%, more preferably at least 70%, even more preferably at least 80%, especially at least 90% or even at least 95% of the methanogenic microorganisms remain viable and/or intact prior to and during the harvesting. This applies especially to the methanogenic microorganisms present in a liquid stream removed from the bioreactor (e.g. in continuous culture). The methanogenic microorganisms may then be recycled back to the bioreactor.

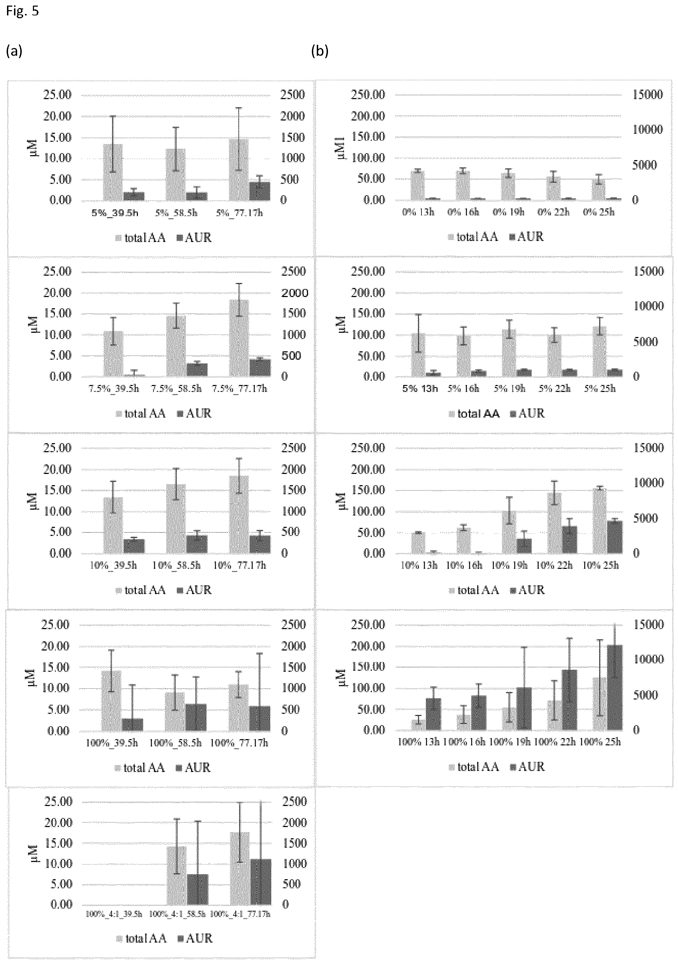

According to another preferred embodiment, the total amino acid concentration in the supernatant of the fermentation broth (used in the harvesting step) is at least 1 μmol/L, preferably at least 5 μmol/L, more preferably at least 10 μmol/L or even at least 25 μmol/L, even more preferably at least 50 μmol/L or even at least 100 μmol/L, especially at least 150 μmol/L (cf. Example 1 and Figure 5). It is particularly preferred when the supernatant has a total amino acid concentration of at least 200 μmol/L, preferably at least 500 μmol/L, especially at least 1000 μmol/L (or even higher lower limits).

The amino acids produced by the methods and uses disclosed herein may be either or both the D- or L-isomer. The amino acid may e.g. be 2-aminobutyrate, alanine, beta-alanine, arginine, aspartate, carnitine, citruline, cystine, dehydroalanine, glutamate, glutamine, glycine, hydroxyproline, isoleucine, leucine, lysine, methionine, norleucine, norvaline, ornithine, phenylalanine, proline, pyroglutamate, pyrroproline, pyrrolysine selenocysteine, selenomethionine, serine, threonine, tryptophan, tyramine, tyrosine, or valine.

The (harvested) amino acids preferably comprise at least one, preferably at least two or even at least three, more preferably at least four or even at least five, even more preferably at least seven or even at least nine, yet more preferably at least 12 or even at least 15, yet even more preferably at least 17 or even at least 18, especially all of the 20 canonical amino acids (i.e. Asp, Glu, Asn, Ser, His, Gln, Gly, Thr, Arg, Ala, Tyr, Val, Met, Trp, Ile, Phe, Leu, Lys, Cys and Pro). It is especially preferred when the (harvested) amino acids comprise one or more of the following: essential amino acids (essential for human consumption), branched chain amino acids (BCAAs) and glutamate, preferably wherein it least 50 mol%, preferably at least 60 mol%, especially at least 70 mol% of the (produced or harvested) amino acids are essential amino acids, branched chain amino acids (BCAAs) or glutamate.

According to another preferred embodiment, the total amino acid production rate per volume of fermentation broth is at least 0.01 $\mu$mol L$^{-1}$h$^{-1}$, preferably at least 0.05 $\mu$mol L$^{-1}$h$^{-1}$, more preferably at least 0.1 $\mu$mol L$^{-1}$h$^{-1}$, even more preferably at least 0.5 $\mu$mol L$^{-1}$h$^{-1}$, yet even more preferably at least 1.0 $\mu$mol L$^{-1}$h$^{-1}$, especially at least 5 $\mu$mol L$^{-1}$h$^{-1}$ or even at least 10 $\mu$mol L$^{-1}$h$^{-1}$ (or even higher) .

According to yet another preferred embodiment, the total amino acid production rate per biomass is at least 0.1 $\mu$mol g$^{-1}$h$^{-1}$, preferably at least 0.5 $\mu$mol g$^{-1}$h$^{-1}$, more preferably at least 1.0 $\mu$mol g$^{-1}$h$^{-1}$, even more preferably at least 5 $\mu$mol g$^{-1}$h$^{-1}$, yet even more preferably at least 10 $\mu$mol g$^{-1}$h$^{-1}$, especially at least 50 $\mu$mol g$^{-1}$h$^{-1}$ or even at least 100 $\mu$mol g$^{-1}$h$^{-1}$ (or even higher) .

The methanogenic microorganisms used may be natural (e.g. natural isolates or laboratory strains obtained therefrom) or genetically manipulated. By way of example, genetic manipulation in methanogenic archeaea such as manipulation based on site-directed mutagenesis, selectable markers, transformation methods, and reporter genes, is available to the skilled person, see e.g., Sarmiento et al. 2011. CRISPR-based gene editing and other CRISPR-based genetic tools are also available to the skilled person, see. e.g. Nayak & Metcalf, 2017 and Dhamad & Lessner, 2020.

According to a particular preferred embodiment, the methanogenic microorganisms comprise archaea selected from any of Methanobacteriales, Methanococcales, Methanomicrobiales, Methanosarcinales, Methanopyrales, Methanocellales, Methanomassiliicoccales, and Methanonatronarchaeales, preferably Methanobacteriales and Methanococcales; more preferably selected from Methanobacteriaceae, Methanocaldococcaceae and Methanococcaceae; in particular selected from *Methanothermobacter, Methanothermococcus* and *Methanococcus.* Particularly preferred are *Methanothermobacter marburgensis, Methanocaldococcus jannaschii, Methanococcus igneus* and *Methanocaldococcus villosus.* Other suitable methanogenic archaea species are for instance: *Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arboriphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanocella arvoryzae, Methanocella conradii, Methanocella paludicola, Methanothermobacter thermautotrophicum, Methanothermobacter thermoflexus, Methanothermobacter thermophilus, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanomicrococcus blatticola, Methanoplanus endosymbiosus, Methanoplanus limicola, Methanoplanus petrolearius, Methanoregula boonei, Methanosaeta concilii, Methanosaeta harundinacea, Methanosaeta pelagica, Methanosaeta thermophila, Methanosarcina acetivorans, Methanosarcina barkeri, Methanosarcina mazei, Methanosarcina thermophila, Methanomicrobium mobile, Methanococcus aeolicus, Methanococcus maripaludis, Methanococcus vannielii, Methanococcus voltae, Methanothermococcus thermolithotrophicus, Methanopyrus kandleri, Methanothermobacter thermautotrophicum, Methanocaldococcus fervens, Methanocaldococcus indicus, Methanocaldococcus infernus,* and *Methanocaldococcus vulcanius.*

Further archaeal species or strains suitable for the present invention are e.g. disclosed in following research articles: Leigh 2000; Fardeu et al. 1987; Belay et al 1984; Murray and Zinder 1984; Blank et al. 1995; Bult et al 1996; Kessler et al, 1997, Mauerhofer et al, 2021; all of them included herein by reference. Strains may e.g. be obtained from the "Deutsche Sammlung für Mikroorganismen und Zellkulturen GmbH" (DSMZ) (Braunschweig, Germany).

The methanogenic microorganisms may be hydrogenotrophic, aceticlastic, methylotrophic (e.g. H$_2$-dependent methylotrophic) or methoxydotrophic.

A (defined) coculture of methanogenic microorganisms in the bioreactor has also turned out to be advantageous. Accordingly, the methanogenic microorganisms (in the bioreactor) preferably comprise at least two different species.

It is highly preferred that the fermentation is started with the methanogenic microorganisms in chemically defined fermentation medium.

In another preferred embodiment, the fermentation is performed under anaerobic conditions.

According to another preferred embodiment, the method is a continuous process, a fed-batch process, a batch process, a closed batch process, a repetitive batch process, a repetitive fed-batch process or a repetitive closed batch process, preferably a continuous process, a fed-batch process or a repetitive fed-batch process, especially a continuous process. It is particularly preferred that the continuous process (culture) is a chemostat process (culture), especially with controlled pH.

When the method is a continuous process (continuous culture), it is preferred that the dilution rate D is 0.001 h$^{-1}$ to 1.5 h$^{-1}$, preferably 0.01 h$^{-1}$ to 0.5 h$^{-1}$, especially 0.0125 h$^{-1}$ to 0.1 h$^{-1}$.

Nitrogen-fixing conditions and/or carbon-fixing conditions have turned out to be advantageous: Thus, according to a further preferment, at least 50%, preferably at least 60%, more preferably at least 70%, even more preferably at least 80%, yet even more preferably at least 90% or even more preferably at least 95%, especially at least 99% or even at least 99.9% of all nitrogen atoms of all nitrogen sources fed to the bioreactor are fed to the bioreactor in the form of nitrogen gas. In yet another preferred embodiment, at least 50%, preferably at least 60%, more preferably at least 70%, even more preferably at least 80%, yet even more preferably at least 90% or even more preferably at least 95%, especially at least 99% or even at least 99.9% of all carbon atoms of all carbon sources fed to the bioreactor are fed to the bioreactor in the form of carbon dioxide gas and/or carbon monoxide gas.

Typically, also a sulfur source is fed to be bioreactor. This source preferably comprises cysteine and/or sulfide, especially wherein the fermentation broth comprises sulfide at a concentration of 0.001 - 150 mg/L, preferably 0.01 - 100 mg/L, especially 1 to 80 mg/L and/or wherein the sulfide feed rate or cysteine feed rate is 0.0001 - 0.2 mol $L^{-1}h^{-1}$, preferably 0.001 - 0.05 mol $L^{-1}h^{-1}$.

A variety of different bioreactors may be used in the methods and uses disclosed herein. Liquid phase bioreactors (e.g. stirred tank, packed bed, one liquid phase, two liquid phase, hollow fiber membrane) are well known in the art. Multiphase bioreactors (e.g., bubble column bioreactor, trickle bed bioreactor (fixed or packed bed), fluidized bed bioreactor) may also be used. The bioreactor is typically partially made of (stainless) steel, plastic and/or glass. Usually, the bioreactor contains at least one inlet, which allows a gas or gas mixture to enter, and at least two outlets. One outlet allows for the removal of a liquid stream, which contains the one or more fermentation products (i.e. amino acids), and the other outlet allows for the removal of a gas stream (such as methane produced by the methanogenic microorganisms). In embodiments, the bioreactor is a chemostat.

There is also an increasing demand for non-proteinogenic amino acids. Norvaline (also called Nva herein) is a non-canonical amino acid which finds increasing use as a dietary supplement. Further, it is used for inducing vasodilation (see for instance US 11,260,039 B1). It was also suggested as a therapeutic agent against Alzheimer's disease (Polis et al, 2019).

Not related thereto, norvaline has also gained interest when found incorporated into protein-based biopharmaceuticals produced by recombinant *E. coli* fermentation processes (Biermann et al, 2013) which is generally not desired.

CN 106520651 A discloses a method for producing L-norvaline through enzymatic transformation.

US 2006/0057685 A1 relates to a fermentative method for producing norvaline using a bacterium of the *Enterobacteriaceae* family having all acetohydroxy acid synthases inactivated.

In the course of the present invention, it was surprisingly observed that methanogenic archaea are capable of norvaline production and active excretion of norvaline into the cell culture supernatant, in particular under carbon-fixing conditions (or carbon- and nitrogen-fixing conditions). Norvaline production has not been observed before in archaea, let alone in methanogenic archaea. This finding of the present invention enables combined carbon (and $N_2$) fixation and concomitant norvaline production.

Thus, in a particularly preferred embodiment, the (harvested) amino acids preferably comprise norvaline.

According to another preferred embodiment, the norvaline production rate per volume of fermentation broth is at least 0.001 $\mu$mol $L^{-1}h^{-1}$, preferably at least 0.005 $\mu$mol $L^{-1}h^{-1}$, more preferably at least 0.01 $\mu$mol $L^{-1}h^{-1}$, even more preferably at least 0.05 $\mu$mol $L^{-1}h^{-1}$, yet even more preferably at least 0.1 $\mu$mol $L^{-1}h^{-1}$, especially at least 0.5 $\mu$mol $L^{-1}h^{-1}$ or even at least 1.0 $\mu$mol $L^{-1}h^{-1}$ (or even higher) .

According to yet another preferred embodiment, the norvaline production rate per biomass is at least 0.01 $\mu$mol $g^{-1}h^{-1}$, preferably at least 0.05 $\mu$mol $g^{-1}h^{-1}$, more preferably at least 0.1 $\mu$mol $g^{-1}h^{-1}$, even more preferably at least 0.5 $\mu$mol $g^{-1}h^{-1}$, yet even more preferably at least 1.0 $\mu$mol $g^{-1}h^{-1}$, especially at least 5 $\mu$mol $g^{-1}h^{-1}$ or even at least 10 $\mu$mol $g^{-1}h^{-1}$ (or even higher).

The present invention further relates to the following embodiments:

Embodiment 1. A method of producing amino acids by fermentation in a bioreactor, wherein the bioreactor comprises methanogenic microorganisms in a fermentation broth, the method comprising at least the step of feeding a gaseous carbon source comprising carbon dioxide and/or carbon monoxide, a nitrogen source comprising nitrogen gas and preferably a sulfur source to the bioreactor under conditions such that the methanogenic microorganisms produce the amino acids, wherein the fermentation broth comprises ammonium at a concentration of at least 0.1 mmol/L and the tungstate concentration in the fermentation broth is below 0.1 $\mu$mol/L, preferably below 0.01 $\mu$mol/L, especially below 0.001 $\mu$mol/L.

Embodiment 2. A method of producing amino acids by fermentation in a bioreactor, wherein the bioreactor comprises methanogenic microorganisms in a fermentation broth, the method comprising at least the step of feeding a gaseous carbon source comprising carbon dioxide and/or carbon monoxide, a nitrogen source comprising nitrogen gas and preferably a sulfur source to the bioreactor under conditions such that the methanogenic microorganisms produce the amino acids; wherein the fermentation broth comprises ammonium at a concentration of at least 0.1 mmol/L and the orthotungstate concentration in the fermentation broth is below 0.1 $\mu$mol/L, preferably below 0.01 $\mu$mol/L,

especially below 0.001 µmol/L.

Embodiment 3. The method of embodiment 1 or 2, wherein the ammonium concentration in the fermentation broth stays within 0.1 mmol/L to 200 mmol/L, preferably 2 mmol/L to 100 mmol/L, more preferably 4 to 40 mmol/L, even more preferably 5 mmol/L to 35 mmol/L, yet even more preferably 6 mmol/L to 30 mmol/L, especially 7 mmol/L to 25 mmol/L, or even 10 mmol/L to 20 mmol/L for at least 5 min, preferably at least 10 min, even more preferably at least at least 20 min, yet even more preferably at least 1 h, especially at least 5 h or even at least 10 h (or at least 20 h or at least 40 h).

Embodiment 4. The method of any one of embodiments 1 to 3, wherein the fermentation broth comprises ammonium at a concentration of 0.1 mmol/L to 200 mmol/L, preferably 2 mmol/L to 100 mmol/L, more preferably 4 to 40 mmol/L, even more preferably 5 mmol/L to 35 mmol/L, yet even more preferably 6 mmol/L to 30 mmol/L, especially 7 mmol/L to 25 mmol/L, or even 10 mmol/L to 20 mmol/L.

Embodiment 5. The method of any one of embodiments 1 to 4, wherein the fermentation broth is essentially free of tungstate.

Embodiment 6. The method of any one of embodiments 1 to 5, wherein the fermentation broth is essentially free of orthotungstate.

Embodiment 7. The method of any one of embodiments 1 to 6, wherein an electron donor compound suitable for the methanogenic microorganisms is fed to the bioreactor.

Embodiment 8. The method of embodiment 7, wherein the electron donor compound is selected from hydrogen gas, acetate, a methyl compound (preferably selected from methylamines, methyl sulfides and methanol), any other alcohol (preferably a secondary alcohol such as 2-propanol or 2-butanol), a methoxylated aromatic compound, formate, and combinations thereof; preferably selected from hydrogen gas, acetate, methanol and combinations thereof.

Embodiment 9. The method of embodiment 7, wherein the electron donor compound comprises hydrogen gas.

Embodiment 10. The method of any one of embodiments 1 to 6, wherein hydrogen gas, acetate, a methyl compound (preferably selected from methylamines, methyl sulfides and methanol), any other alcohol (preferably a secondary alcohol such as 2-propanol or 2-butanol), a methoxylated aromatic compound and/or formate are fed to the bioreactor; preferably wherein hydrogen gas, acetate, methanol or combinations thereof are fed to the bioreactor.

Embodiment 11. The method of embodiment 10, wherein hydrogen gas is fed to the bioreactor.

Embodiment 12. The method of embodiment 10 or 11, wherein acetate and methanol are fed to the bioreactor.

Embodiment 13. The method of any one of embodiments 1 to 12, further comprising the step of harvesting at least a portion of the amino acids from the bioreactor.

Embodiment 14. The method of embodiment 13, wherein the harvesting comprises removing a liquid stream from the bioreactor, wherein the liquid stream comprises fermentation broth, methanogenic microorganisms, and produced amino acids (in particular in the supernatant), separating the methanogenic microorganisms from the liquid stream and recycling the methanogenic microorganisms back to the bioreactor; preferably wherein at least 50%, preferably at least 60%, more preferably at least 70%, even more preferably at least 80%, especially at least 90% or even at least 95% of the methanogenic microorganisms remain viable and/or intact.

Embodiment 15. The method of embodiment 14, further comprising the step of purifying the produced amino acids from the liquid stream, in particular from the supernatant of the fermentation broth; preferably wherein the total produced amino acid concentration in the supernatant of the fermentation broth is at least 1 µmol/L, preferably at least 5 µmol/L, more preferably at least 10 µmol/L or even at least 25 µmol/L, even more preferably at least 50 µmol/L or even at least 100 µmol/L, especially at least 150 µmol/L.

Embodiment 16. The method of any one of embodiments 1 to 15, further comprising harvesting at least a portion of the amino acids from the supernatant of the fermentation broth.

Embodiment 17. The method of any one of embodiments 1 to 16, wherein the supernatant of the fermentation broth has a total protein content of less than 1000 $\mu$g/mL, preferably less than 500 $\mu$g/mL, more preferably less than 250 $\mu$g/mL, even more preferably less than 100 $\mu$g/mL, yet even more preferably less than 50 $\mu$g/ml or even less than 40 $\mu$g/mL, especially less than 30 $\mu$g/mL or even less than 20 $\mu$g/mL.

Embodiment 18. The method of any one of embodiments 1 to 17, wherein at least 50%, preferably at least 60%, more preferably at least 70%, even more preferably at least 80%, especially at least 90% or even at least 95% of the methanogenic microorganisms remain viable and/or intact prior to and/or during the harvesting.

Embodiment 19. The method of any one of embodiments 1 to 18, wherein the total amino acid concentration in the supernatant of the fermentation broth is at least 1 $\mu$mol/L, preferably at least 5 $\mu$mol/L, more preferably at least 10 $\mu$mol/L or even at least 25 $\mu$mol/L, even more preferably at least 50 $\mu$mol/L or even at least 100 $\mu$mol/L, especially at least 150 $\mu$mol/L.

Embodiment 20. The method of any one of embodiments 1 to 19, wherein the amino acids further comprise, or wherein said portion further comprises, at least one, preferably at least two or even at least three, more preferably at least four or even at least five, even more preferably at least seven or even at least nine, yet more preferably at least 12 or even at least 15, yet even more preferably at least 17 or even at least 18, especially all of the 20 canonical amino acids (in particular their L-form).

Embodiment 21. The method of any one of embodiments 1 to 19, wherein the amino acids comprises, or wherein said portion comprises, at least one, preferably at least two or even at least three, more preferably at least four or even at least five, even more preferably at least seven or even at least nine, yet more preferably at least 12 or even at least 15, yet even more preferably at least 17 or even at least 18, especially all of: 2-aminobutyrate, alanine, beta-alanine, arginine, aspartate, carnitine, citruline, cystine, dehydroalanine, glutamate, glutamine, glycine, hydroxyproline, isoleucine, leucine, lysine, methionine, norleucine, norvaline, ornithine, phenylalanine, proline, pyroglutamate, pyrroproline, pyrrolysine selenocysteine, selenomethionine, serine, threonine, tryptophan, tyramine, tyrosine, and valine (in particular their L-form).

Embodiment 22. The method of any one of embodiments 1 to 19, wherein the amino acids comprise, or wherein said portion comprises, at least one, preferably at least two or even at least three, more preferably at least four or even at least five, even more preferably at least seven or even at least nine, yet more preferably at least 12 or even at least 15, yet even more preferably at least 17 or even at least 18, especially all of: 2-aminobutyrate, alanine, beta-alanine, arginine, aspartate, carnitine, citruline, cystine, dehydroalanine, glutamate, glutamine, glycine, hydroxyproline, isoleucine, leucine, lysine, methionine, norleucine, norvaline, ornithine, phenylalanine, proline, pyroglutamate, pyrroproline, pyrrolysine selenocysteine, selenomethionine, serine, threonine, tryptophan, tyramine, tyrosine, and valine (in particular their L-form).

Embodiment 23. The method of any one of embodiments 1 to 19, wherein the amino acids comprise, or wherein said portion comprises, one or more of the following: essential amino acids (essential for human consumption), branched chain amino acids (BCAAs) and glutamate (in particular their L-form); preferably wherein it least 50 mol%, preferably at least 60 mol%, especially at least 70 mol% of the (produced or harvested) amino acids are essential amino acids, branched chain amino acids (BCAAs) or glutamate (in particular their L-form).

Embodiment 24. The method of any one of embodiments 1 to 23, wherein the production rate of said amino acids per volume of fermentation broth is at least 0.01 $\mu$mol L$^{-1}$h$^{-1}$, preferably at least 0.05 $\mu$mol L$^{-1}$h$^{-1}$, more preferably at least 0.1 $\mu$mol L$^{-1}$h$^{-1}$, even more preferably at least 0.5 $\mu$mol L$^{-1}$h$^{-1}$, yet even more preferably at least 1.0 $\mu$mol L$^{-1}$h$^{-1}$, especially at least 5 $\mu$mol L$^{-1}$h$^{-1}$ or even at least 10 $\mu$mol L$^{-1}$h$^{-1}$.

Embodiment 25. The method of any one of embodiments 1 to 24, wherein the total amino acid production rate per volume of fermentation broth is at least 0.01 $\mu$mol L$^{-1}$h$^{-1}$, preferably at least 0.05 $\mu$mol L$^{-1}$h$^{-1}$, more preferably at least 0.1 $\mu$mol L$^{-1}$h$^{-1}$, even more preferably at least 0.5 $\mu$mol L$^{-1}$h$^{-1}$, yet even more preferably at least 1.0 $\mu$mol L$^{-1}$h$^{-1}$, especially at least 5 $\mu$mol L$^{-1}$h$^{-1}$ or even at least 10 $\mu$mol L$^{-1}$h$^{-1}$.

Embodiment 26. The method of any one of embodiments 1 to 25, wherein the production rate of said amino acids per biomass is at least 0.1 $\mu$mol g$^{-1}$h$^{-1}$, preferably at least 0.5 $\mu$mol g$^{-1}$h$^{-1}$, more preferably at least 1.0 $\mu$mol g$^{-1}$h$^{-1}$, even more preferably at least 5 $\mu$mol g$^{-1}$h$^{-1}$, yet even more preferably at least 10 $\mu$mol g$^{-1}$ h$^{-1}$, especially at least 50 $\mu$mol g$^{-1}$h$^{-1}$ or even at least 100 $\mu$mol g$^{-1}$h$^{-1}$.

Embodiment 27. The method of any one of embodiments 1 to 26, wherein the total amino acid production rate per biomass is at least 0.1 $\mu$mol g$^{-1}$h$^{-1}$, preferably at least 0.5 $\mu$mol g$^{-1}$h$^{-1}$, more preferably at least 1.0 $\mu$mol g$^{-1}$h$^{-1}$, even more preferably at least 5 $\mu$mol g$^{-1}$h$^{-1}$, yet even more preferably at least 10 $\mu$mol g$^{-1}$ h$^{-1}$, especially at least 50 $\mu$mol g$^{-1}$h$^{-1}$ or even at least 100 $\mu$mol g$^{-1}$h$^{-1}$.

Embodiment 28. The method of any one of embodiments 1 to 27, wherein methane (produced by the methanogenic microorganisms) is harvested from the bioreactor.

Embodiment 29. The method of any one of embodiments 1 to 28, wherein the methanogenic microorganisms are genetically manipulated microorganisms.

Embodiment 30. The method of any one of embodiments 1 to 28, wherein the methanogenic microorganisms are natural isolates or laboratory strains obtained therefrom.

Embodiment 31. The method of any one of embodiments 1 to 30, wherein the methanogenic microorganisms comprise archaea.

Embodiment 32. The method of embodiment 31, wherein the archaea are selected from any of Methanobacteriales, Methanococcales, Methanomicrobiales, Methanosarcinales, Methanopyrales, Methanocellales, Methanomassiliicoccales, and Methanonatronarchaeales, preferably Methanobacteriales and Methanococcales; more preferably selected from Methanobacteriaceae, Methanocaldococcaceae and Methanococcaceae; in particular selected from *Methanothermobacter, Methanothermococcus, Methanocaldococcus* and *Methanococcus.*

Embodiment 33. The method of embodiment 31, wherein the archaea are selected from *Methanothermobacter marburgensis, Methanocaldococcus jannaschii, Methanococcus igneus* and *Methanocaldococcus villosus.*

Embodiment 34. The method of embodiment 31, wherein the archaea are of the genus *Methanothermobacter,* in particular of the species *Methanothermobacter marburgensis.*

Embodiment 35. The method of embodiment 31, wherein the archaea are selected from *Methanothermobacter marburgensis, Methanocaldococcus jannaschii, Methanococcus igneus, Methanocaldococcus villosus, Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arboriphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanocella arvoryzae, Methanocella conradii, Methanocella paludicola, Methanothermobacter thermautotrophicum, Methanothermobacter thermoflexus, Methanothermobacter thermophilus, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanomicrococcus blatticola, Methanoplanus endosymbiosus, Methanoplanus limicola, Methanoplanus petrolearius, Methanoregula boonei, Methanosaeta concilii, Methanosaeta harundinacea, Methanosaeta pelagica, Methanosaeta thermophila, Methanosarcina acetivorans, Methanosarcina barkeri, Methanosarcina mazei, Methanosarcina thermophila, Methanomicrobium mobile, Methanococcus aeolicus, Methanococcus maripaludis, Methanococcus vannielii, Methanococcus voltae, Methanothermococcus thermolithotrophicus, Methanopyrus kandleri, Methanothermobacter thermautotrophicum, Methanocaldococcus fervens, Methanocaldococcus indicus, Methanocaldococcus infernus, Methanocaldococcus vulcanius* and combinations thereof.

Embodiment 36. The method of any one of embodiments 1 to 35, wherein the methanogenic microorganisms comprise at least two different species; preferably wherein at least one, especially at least two, of the species is archaeal, in particular selected from any of Methanobacteriales, Methanococcales, Methanomicrobiales, Methanosarcinales, Methanopyrales, Methanocellales, Methanomassiliicoccales, and Methanonatronarchaeales, preferably Methanobacteriales and Methanococcales, more preferably selected from Methanobacteriaceae, Methanocaldococcaceae and Methanococcaceae, in particular selected from *Methanothermobacter, Methanothermococcus, Methanocaldococcus* and *Methanococcus.*

Embodiment 37. The method of any one of embodiments 1 to 36, wherein the methanogenic microorganisms comprise hydrogenotrophic, aceticlastic, methylotrophic or methoxydotrophic microorganisms or combinations thereof.

Embodiment 38. The method of any one of embodiments 1 to 37, wherein the fermentation is started with methanogenic microorganisms in chemically defined fermentation medium.

Embodiment 39. The method of any one of embodiments 1 to 38, wherein the fermentation is performed under anaerobic conditions.

Embodiment 40. The method of any one of embodiments 1 to 39, wherein the method is a continuous process, a fed-batch process, a batch process, a closed batch process, a repetitive batch process, a repetitive fed-batch process or a repetitive closed batch process, preferably a continuous process, a fed-batch process or a repetitive fed-batch process, especially a continuous process.

Embodiment 41. The method of embodiment 40, wherein the method is a chemostat process.

Embodiment 42. The method of embodiment 40 or 41, wherein the dilution rate D is $0.001 \text{ h}^{-1}$ to $1.5 \text{ h}^{-1}$, preferably $0.01 \text{ h}^{-1}$ to $0.5 \text{ h}^{-1}$, especially $0.0125 \text{ h}^{-1}$ to $0.1 \text{ h}^{-1}$.

Embodiment 43. The method of any one of embodiments 1 to 42, wherein at least 50%, preferably at least 60%, more preferably at least 70%, even more preferably at least 80%, yet even more preferably at least 90% or even more preferably at least 95%, especially at least 99% or even at least 99.9% of all nitrogen atoms of all nitrogen sources fed to the bioreactor are fed to the bioreactor in the form of nitrogen gas.

Embodiment 44. The method of any one of embodiments 1 to 43, wherein at least 50%, preferably at least 60%, more preferably at least 70%, even more preferably at least 80%, yet even more preferably at least 90% or even more preferably at least 95%, especially at least 99% or even at least 99.9% of all carbon atoms of all carbon sources fed to the bioreactor are fed to the bioreactor in the form of carbon dioxide gas and/or carbon monoxide gas.

Embodiment 45. The method of any one of embodiments 1 to 44, wherein the sulfur source is fed to the bioreactor, preferably wherein the sulfur source comprises cysteine and/or sulfide, especially wherein the fermentation broth comprises sulfide at a concentration of 0.001 - 150 mg/L, preferably 0.01 - 100 mg/L, especially 1 to 80 mg/L and/or wherein the sulfide feed rate or cysteine feed rate is $0.0001 - 0.2 \text{ mol L}^{-1}\text{h}^{-1}$, preferably $0.001 - 0.05 \text{ mol L}^{-1}\text{h}^{-1}$.

Embodiment 46. The method of any one of embodiments 1 to 45, wherein the bioreactor is a liquid phase or multiphase bioreactor.

Embodiment 47. The method of any one of embodiments 1 to 46, wherein the bioreactor is any one of stirred tank, packed bed, one liquid phase, two liquid phase, hollow fiber membrane, bubble column, trickle bed or fluidized bed bioreactor.

Embodiment 48. The method of any one of embodiments 1 to 47, wherein the bioreactor is a chemostat.

Embodiment 49. The method of any one of embodiments 1 to 48, wherein the amino acids comprise, or wherein said portion comprises, norvaline, in particular L-norvaline.

Embodiment 50. The method of any one of embodiments 1 to 49, wherein the norvaline (in particular L-norvaline) production rate per volume of fermentation broth is at least $0.001 \text{ } \mu\text{mol L}^{-1} \text{ h}^{-1}$, preferably at least $0.005 \text{ } \mu\text{mol L}^{-1}\text{h}^{-1}$, more preferably at least $0.01 \text{ } \mu\text{mol L}^{-1}\text{h}^{-1}$, even more preferably at least $0.05 \text{ } \mu\text{mol L}^{-1}\text{h}^{-1}$, yet even more preferably at least $0.1 \text{ } \mu\text{mol L}^{-1}\text{h}^{-1}$, especially at least $0.5 \text{ } \mu\text{mol L}^{-1}\text{h}^{-1}$ or even at least $1.0 \text{ } \mu\text{mol L}^{-1} \text{ h}^{-1}$.

Embodiment 51. The method of any one of embodiments 1 to 50, wherein the norvaline (in particular L-norvaline) production rate per biomass is at least $0.01 \text{ } \mu\text{mol g}^{-1}\text{h}^{-1}$, preferably at least $0.05 \text{ } \mu\text{mol g}^{-1}\text{h}^{-1}$, more preferably at least $0.1 \text{ } \mu\text{mol g}^{-1}\text{h}^{-1}$, even more preferably at least $0.5 \text{ } \mu\text{mol g}^{-1}\text{h}^{-1}$, yet even more preferably at least $1.0 \text{ } \mu\text{mol g}^{-1}\text{h}^{-1}$, especially at least $5 \text{ } \mu\text{mol g}^{-1}\text{h}^{-1}$ or even at least $10 \text{ } \mu\text{mol g}^{-1}\text{h}^{-1}$.

Embodiment 52. Use of methanogenic microorganisms for producing amino acids from an electron donor compound, a gaseous carbon source comprising carbon dioxide and/or carbon monoxide, a nitrogen source comprising nitrogen gas and preferably a sulfur source, wherein the fermentation broth comprises ammonium at a concentration of at least 0.1 mmol/L and the tungstate concentration (or the orthotungstate concentration) in the fermentation broth is below 0.1 $\mu$mol/L, preferably below 0.01 $\mu$mol/L, especially below 0.001 $\mu$mol/L.

Embodiment 53. The use of embodiment 52, wherein the fermentation broth is as defined in any one of embodiments 2 to 6, 17, 19 and 45.

Embodiment 54. The use of embodiment 52 or 53, wherein the electron donor compound is as defined in any one of embodiments 7 to 9.

Embodiment 55. The use of any one of embodiments 52 to 54, wherein the amino acids are as defined in any one of embodiments 20 to 23 and 49.

Embodiment 56. The use of any one of embodiments 52 to 55, wherein the production rate is as defined in any one of embodiments 24 to 27, 50 and 51.

Embodiment 57. The use of any one of embodiments 52 to 56, wherein the methanogenic microorganisms are as defined in any one of embodiments 29 to 37.

Embodiment 58. A fermentation broth comprising methanogenic microorganisms, wherein the fermentation broth comprises ammonium at a concentration of at least 0.1 mmol/L and the tungstate concentration (or the orthotungstate concentration) in the fermentation broth is below 0.1 $\mu$mol/L, preferably below 0.01 $\mu$mol/L, especially below 0.001 $\mu$mol/L.

Embodiment 59. The fermentation broth of embodiment 58, wherein the fermentation broth further is as defined in any one of embodiments 2 to 6, 17, 19 and 45.

Embodiment 60. The fermentation broth of embodiment 58 or 59, further comprising an electron donor compound, preferably as defined in any one of embodiments 7 to 9, a carbon source, a nitrogen source and preferably a sulfur source.

Embodiment 61. The fermentation broth of any one of embodiments 58 to 60, wherein the fermentation broth further comprises amino acids, especially in the supernatant; preferably wherein the amino acids are as defined in any one of embodiments 20 to 23 and 49.

Embodiment 62. The fermentation broth of any one of embodiments 58 to 51, wherein the methanogenic microorganisms are as defined in any one of embodiments 29 to 37.

Embodiment 63. A bioreactor comprising the fermentation broth of any one of embodiments 58 to 62.

The present invention is further illustrated by the following figures and examples, without being restricted thereto.

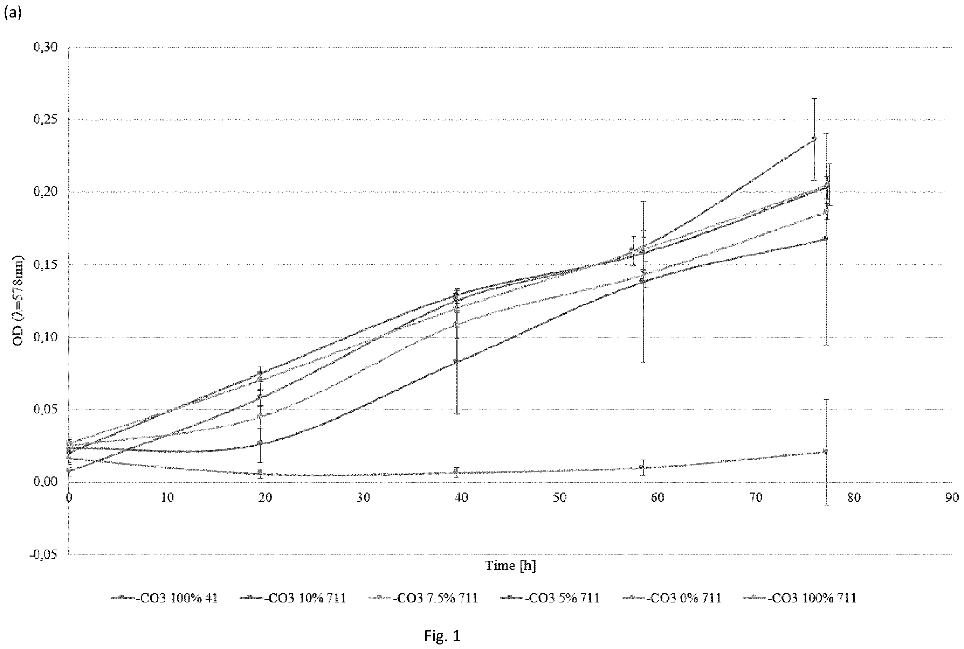

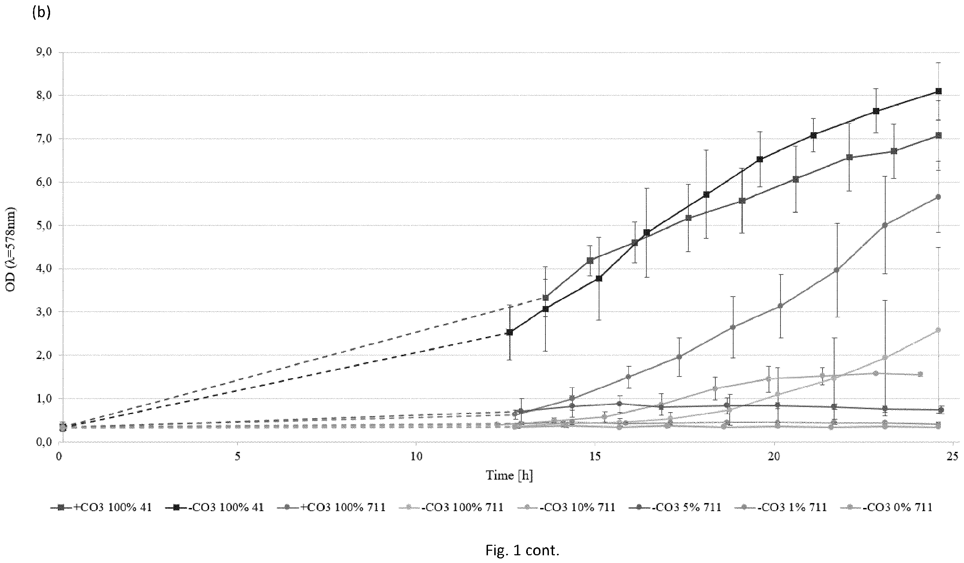

**Figure** 1: Growth kinetics $OD_{578nm}$ of *M. marburgensis* with varying $NH_4^+$ concentrations in relation to original medium gassed with $H_2/CO_2/N_2$ in the ratio 7:1:1 (711). Gassing in the ratio 4:1 (41) served as positive control. (a) Closed batch experiments with one washing step and gas samples taken after 40, 59 and 77 h. (b) Fed-batch experiments without washing. Dashed lines represent growth overnight. +/- indicates the presence of carbon in the media. 100% Reference runs were gassed with $H_2/CO_2$ in the ratio 4:1 (square). Gas samples were taken after approximately 0, 13, 16, 19, 22 and 25 h.

**Figure 2:** $NH_4^+$ concentrations in $\mu$M from (a) closed batch and (b) fed-batch experiments. Colours indicate the different concentrations of $NH_4^+$ in the media, 0% (orange), 5% (red), 7.5% (brown), 10% (yellow), 100% (grey), and 100% with carbon or 4:1 gassing (blue).

**Figure 3:** Amino acid excretion of closed batch 5%, 7.5%. 10%, 100% and 0% experiments. Glutamic acid excretion was dominating.

**Figure 4:** Amino acid excretion of fed-batch 5%, 10%, 100% and 0% experiments. Alanine excretion is dominating.

**Figure 5:** Total amount of excreted amino acids (total amino acids, AA) and ammonium uptake rate (AUR) of closed batch (a) and fed-batch (b) experiments. The amount of excreted AA is increasing with uptake of ammonium.

**Examples**

**Example 1: Amino acid production and active secretion by *Methanothermobacter marburgensis* under $N_2$-fixing conditions**

The aim of our research was to examine the physiological and biotechnological characteristics of biological $N_2$ fixation in connection to $H_2/CO_2$ utilization of different methanogens.

Among several methanogens analyzed, *Methanothermobacter marburgenis* was prioritized to investigate $N_2$ fixation, $CH_4$ production, and amino acid excretion characteristics in closed batch and fed-batch cultivation modes and at different $NH_4^+$ concentrations.

In brief, *M. marburgensis* was grown on chemically defined minimal medium with different concentrations of ammonium chloride ($NH_4Cl$) in a $N_2/H_2/CO_2$ atmosphere. This enabled the quantification of ammonia uptake, $N_2$ fixation, amino acid excretion and the conversion of $H_2/CO_2$ to $CH_4$. $N_2$ fixation by *M. marburgensis* was be confirmed in all experiments with $H_2/N_2/CO_2$ in the gas phase. Furthermore, the active excretion of proteinogenic amino acids was found, with highest detected values of glutamic acid, alanine, glycine and asparagin. The highest general production of 7.5 $\mu$mol L$^{-1}$h$^{-1}$ was detected under "100%" $NH_4^+$ concentration (see Table 1 below) in closed batch after 40 h. Hence, the concomitant production of amino acids and $CH_4$ from $CO_2$ turned out to be of biotechnological relevance in an integrated approach coupling biomethanation and $N_2$ fixation in a biorefinery concept.

Materials and methods

Strains

The following strains were selected for experiments: *Methanothermobacter marburgensis* (Schönheit et al. 1980; Wasserfallen et al. 2000), *Methanobacterium thermaggregans* (Blotevogel and Fischer 1985), *Methanococcus maripaludis* (Jones et al. 1983), *Methanocaldococcus villosus* (Bellack et al. 2011) and *Methanothermococcus okinawensis* (Takai et al. 2000). These strains may e.g. be obtained from the DSMZ, Braunschweig, Germany.

Media

Pre-cultures of *M. villosus* and *M. okinawensis,* were grown on chemically defined medium according to Taubner & Rittmann, 2016. *M. maripaludis* was grown in McN medium (cf. Mauerhofer et al. 2021). *Methanobacterium thermaggregans* and *Methanothermobacter marburgensis* were cultivated on MM medium.

MM Medium (see also Table 1 below): $NH_4Cl$ 2.1 g/L and $KH_2PO_4$ 6.8 g/L in dd$H_2O$. 200x trace element (TE) solution to be added (e.g. up to a concentration of 5 mL per L of MM medium): Titriplex I 9 g/L, add 800 mL $H_2O$ and adjust the pH to 6.5 with 5 mol/L NaOH solution, then add (up to target concentration): $MgCl_2 \cdot 6H_2O$ 8 g/L, $FeCl_2 \cdot 4H_2O$ 2 g/L, $COCl_2 \cdot 6H_2O$ 40 mg/L, $NiCl_2 \cdot 6H_2O$ 240 mg/L, $NaMoO_4 \cdot 2H_2O$ 40 mg, then adjust to pH 7.0 and volume of 1 L with 1 mol/L NaOH and dd$H_2O$. The medium may further contain e.g. 3.6 g/L $NaHCO_3$ as a carbon source. As a sulfur source, e.g. 2 mL of 0.5 mol/L $Na_2S \cdot 9H_2O$ per liter may be added after anaerobization and autoclaving.

Surprisingly, it turned out that it was advantageous that the TE solution (and consequently, the medium) did not contain tungstate (or, at least, only below 0.1 $\mu$mol/L, preferably below 0.01 $\mu$mol/L, especially below 0.001 $\mu$mol/L), as amino acid yield under nitrogen fixing conditions was lower otherwise.

Medium was aliquoted into 117 mL serum bottles (VWR, Austria) to a total working volume of 50 mL, closed with blue rubber stoppers (pre-boiled for ten times 30 min, 20 mm, butyl rubber, Chemglass Life Sciences) and aluminum crimp caps (Ochs Laborbedarf, Bovenden, Germany). Sterile L-Cysteine-HCl·$H_2O$, sterile $NaHCO_3$ solution and $Na_2S \cdot 9H_2O$ according to the media composition were added after autoclaving in an anaerobic glove box (Coy Laboratory Products, Grass Lake, USA). To ensure conditions are anaerobic the atmosphere in the headspace was changed by vacuuming and gassing with the respective gas ($H_2/CO_2$ or $H_2/CO_2/N_2$) mixture up to 2 bar rel. (3 bar abs.) repeating the procedure five times (Taubner & Rittmann 2016). For gassing, sterile syringe filters (w/0.2c $\mu$m cellulose, VWR International, USA) and sterile needles (disposal hypodermic needle, Gr 14, 0.60 $\times$ 30 mm, 23 G $\times$ 1 1/4", Braun, Germany) were used.

Nitrogen-free (N-free) media were prepared by omitting $NH_4Cl$ (2.1 g L$^{-1}$) and replace it with a chemically equal molar amount of NaCl (2.3 g L$^{-1}$) to ensure the correct salt concentration in the medium. To replace L-cysteine monohydrate, a diluted HCl solution was used to retrieve the pH value. MM medium with varying $NH_4^+$ concentrations, was made as shown in Table 1. To ensure that $CO_2$ was the only carbon source, $Na_2CO_3$ was replaced by equal molarities of NaCl. Medium without $Na_2CO_3$ was manually adjusted to pH 6.8 by titrating 10 mol L$^{-1}$ NaOH.

**Table 1:** Composition of MM medium with varying NH4+ concentration*

| g L$^{-1}$ | 100% + | 100% - | 50% - | 25% - | 10% - | 5% - | 1% - | 0% - |
|---|---|---|---|---|---|---|---|---|
| KH$_2$PO$_4$ | 6.8 | 6.8 | 6.8 | 6.8 | 6.8 | 6.8 | 6.8 | 6.8 |
| Na$_2$CO$_3$ | 3.6 | 0 | 0 | 0 | 0 | 0 | 0 | 0 |
| NH$_4$Cl | 2.1 | 2.1 | 1.05 | 0.525 | 0.21 | 0.105 | 0.021 | 0 |
| NaCl mL L$^{-1}$ | 0 | 2 | 2.15 | 2.725 | 3.07 | 3.185 | 3.277 | 3.3 |
| TE 200x | 5 | 5 | 5 | 5 | 5 | 5 | 5 | 5 |

*in relation to original MM medium in g L$^{-1}$. Percentage of NH$_4$Cl in relation to original MM medium (which already contains 2.1 g L$^{-1}$ NH$_4$Cl). -/+ presents the absence and presence of Na$_2$CO$_3$. TE: trace element solution

Chemicals

H$_2$ (99.999%), CO$_2$ (99.999%), N$_2$ (99.999%), H$_2$/CO$_2$ (80%/20%), H$_2$/CO$_2$/N$_2$ (77.74%/11.13%/11.13%) were used for closed batch and fed-batch experiments. For gas chromatography (GC), N$_2$/CO$_2$ (80%/20%), CH$_4$ (99.995%) and the standard test gas (Messer GmbH, Wien, Austria) (containing 0.01 Vol.-% CH$_4$, 0.08 Vol.-% CO$_2$ in N$_2$) was additionally used. All gases, except the standard test gas, were purchased from Air Liquide (Air Liquide GmbH, Schwechat, Austria). All other chemicals were of highest grade available.

Closed batch experiments

Cultures were incubated in a water bath (Burgwedel, Germany) at 65°C (*M. marburgensis, M. thermaggregans* and *M. okinawensis*) or in a shaking air incubator at 37°C (*M. maripaludis*) (Burgwedel, Germany) and 80°C (*M. villosus*) (LABWIT Scientific Pty Ltd, Australia). For the purpose of N$_2$ fixation, all closed batch experiments were performed in a H$_2$/N$_2$/CO$_2$ atmosphere. For selecting the strain for prioritization, *M. marburgensis, M. maripaludis, M. thermaggregans, M. villosus* and *M. okinawensis* were grown in triplicates (n = 3) with one zero control to an OD$_{578}$ of approximately 0.7. As *M. marburgensis* had one of the highest NH$_4^+$ concentrations in the media, it was additionally examined if growth was affected by reducing the amount of NH$_4^+$ to one tenth of the original media concentration. To remove residual nitrogenous compounds from the media, pre-culture cells were washed before inoculation. For a complete N-free media all cultures were washed three times, for all other experiments one or no time. Experiments of only *M. marburgensis* were performed in quadruplicates (n = 4) or octuplicates (n = 8) at different NH$_4^+$ concentrations ("0%", "1%", "10%", "25%", "50%" and "100%", cf. Table 1 above) in relation to original media composition of 2.1 g L$^{-1}$. A pre-culture with one tenth of NH$_4^+$ served as inoculum. The batch with 0% served as negative control and 100% gassed with H$_2$/CO$_2$ in the ratio 4:1 as positive control.

After every incubation time the serum bottles were left at room temperature for 45 min to cool down. Pressure was measured with a digital manometer (Keller GmbH, Winterthur, Switzerland). Growth was measured spectrophotometrically via OD ($\lambda$ = 578 nm, blanked with Milli-Q water) (Beckman Coulter, California, USA). Liquid samples of 1 mL were taken and centrifuged at full speed (13200 rpm) for 30 min. Cell pellets and supernatant of each experiment were stored in sterile Eppendorf tubes until further analysis at -20°C.

Fed-batch experiments

All fed-batch experiments were performed with M. *marburgensis* in triplicates with H$_2$/CO$_2$/N$_2$ gassing at a ratio of 7:1:1 in a DASGIP® 2.2 L bioreactor system (SR1500ODLS, Eppendorf AG, Hamburg, Germany) with 1.5 L working volume of MM medium including 100 $\mu$l L$^{-1}$ of antifoam (Struktol SB2023, Schill und Seilacher, Hamburg, Germany). Best growth conditions are pH of 7 and a temperature of 65°C. Gassing of N$_2$ and CO$_2$ was controlled via the MX4/4 unit (Eppendorf AG, Hamburg, Germany). H$_2$ gas flow was controlled via the C100L Unit (Sierra Instruments, Monterey, USA). Gassing was performed with the same ratio as closed batch experiments. Redox potentials and pH values were monitored by individual redox- and pH-probes (Mettler Toledo GmbH, Wien, Austria). Every fed-batch cultivation was performed with the inoculum of a stock culture of *M. marburgensis,* adapted to fed-batch cultivation. Before inoculation, the bioreactor was gassed with H$_2$/N$_2$/CO$_2$ to ensure conditions are anaerobic and 5 mL of 0.5 mol L$^{-1}$ Na$_2$S·9H$_2$O were added. Immediately after inoculation of 30 mL, feeding of 0.2 mL h$^{-1}$ 0.5M Na$_2$S·9H$_2$O was started and the agitation speed was set to 1600 rpm. Gaseous samples were taken after approximately 0, 13, 16, 19, 22 and 25 h. Batches with and without Na$_2$CO$_3$ in the media, performed under H$_2$/CO$_2$ atmosphere (ratio 4:1) served as reference. For analyzing growth and amino acid excretion, liquid samples were taken and treated as described above.

Ammonium determination

$NH_4^+$ determination was performed using a modified procedure according to the method described before (Kandeler 1988). The oxidation solution, the colour reagent and the $NH_4Cl$ stock solution were prepared freshly before the measurement. As standards, nine different concentrations, ranging from 100 $\mu$mol $L^{-1}$ to 1000 $\mu$mol $L^{-1}$ of $NH_4Cl$, were prepared. Samples were diluted with MilliQ to an end concentration between the standard ranges. Before the measurement, 300 $\mu$L of colour reagent and 120 $\mu$L oxidation solution were added immediately to the standards and samples and shortly mixed. After 30 min in the dark, measurement ($\lambda$ = 660nm) was performed using a 96 well plate (Microtest Plate 96 Well, F, Sarstedt AG & C0, Nümbrecht, Germany) with a plate photometer (Sunrise plate reader, Tecan Group AG, Männedorf, Switzerland). Regression curve $R^2$ was always higher than 0.999.

Gas chromatography

Closed batch experiments and the off-gas composition ($H_2$, $CO_2$, $CH_4$ and $N_2$) of the collected gas samples from fed-batch experiments were analyzed by using the Agilent Gas Chromatograph (Agilent 7890A GC, Agilent Technologies, Santa Clara, CA, USA) equipped with a thermal conductivity detector (TDC) and a 19808 Shin Carbon ST Micropacked Column (Restek GmbH, Bad Homburg, Germany).

Amino acid analysis

For amino acid analyses, supernatant of samples (obtained as described above) were diluted with Mill-Q water at the ratio of 1:4. Measurements were performed on Agilent 1260 Infinity Bioinert HPLC system containing a fluorescence detector, a column oven, an autosampler and a quaternary pump. 1 mL of sample was mixed with 75 $\mu$L borate buffer (0.4 N in water, pH = 10.2; Agilent Technologies) followed by 5 $\mu$L OPA reagent, (3-mercaptopropionic acid in 0.4 mol $L^{-1}$ borate buffer and 10 mg $mL^{-1}$ of o-phthalaldehyde (OPA); Agilent Technologies). 100 $\mu$L of the mixture was injected to HPLC system after 2 min at 27 °C. Fluorescent derivates (primary dissolved free amino acids) were separated at 25 °C and a flow rate of 0.8 mL $min^{-1}$ on a Zorbax ECLIPSE AAA column (4.6 × 150 mm, 3.5 $\mu$m particle size, Agilent Technologies) with a Zorbax ECLIPSE AAA guard cartridge (4.6 × 150 mm, 5 $\mu$m particle size, Agilent Technologies). Excitation wavelength was 340 nm and emission 450 nm. The use of a gain factors 9 or 10 was depended on the expected concentration and pre-tested before. For identification and quantification of peaks a primary amino acid standard mix (AAS18, Sigma Aldrich) in different concentrations was prepared for each run according to the concentration range of the samples (100 nmol $L^{-1}$ to 15 $\mu$mol $L^{-1}$). AAS18 standard mix lacks five amino acids (asparagine (Asn), glutamic acid (Glu), gamma-aminobutyric acid (GABA), taurine (Tau), tryptophane (Trp); Sigma Aldrich) which were added. In total 20 different AA could be measured with this method. Valine and Methionine were excluded from evaluation as they are located within a signal noise "ammonium peak" and therefore hard to measure within experiments with high $NH_4^+$ concentrations. The details of this method were as published before (Taubner et al. 2019).

GC analysis

The relative pressure in bar within the serum bottle was measured with a digital manometer (Keller GmbH, Winterthur, Switzerland). Gaseous substance (n /mol) in the serum bottles headspace was calculated via the ideal gas law. Headspace volume was determined in earlier experiments and adjusted after every OD measurement by the extracted sample volume of 0.75 mL. All measurements were performed at room temperature (25°C).

To obtain the actual amount of $N_2$ the pressure inside the serum bottles was multiplied by 0.11392 based on the exact percentage of $N_2$ (11.392 Vol.-%) in the gas mixture, then multiplied with the normalized gas composition gained from GC measurement. The values of the zero-control served as $N_2$ baseline. Molecular $N_2$ uptake rate (NUR / mmol $L^{-1}h^{-1}$) was calculated by dividing the deviation of $N_2$ before and after incubation ($\Delta N_2$) by volume of the liquid medium and the time since last incubation ($\Delta t$):

$$NUR \left[ \frac{mmol}{L \cdot h} \right] = \frac{N_2 [mol]}{Vol.(med)[L] \cdot t[h]}$$

The quantitative/specific nitrogen uptake ($qN_2$ / mmol $h^{-1}$ $g^{-1}$) was determined by dividing the NUR by the biomass concentration (x / g $L^{-1}$) calculated with an experimentally determined coefficient:

$$qN_2\left[\frac{mmol}{h\cdot g}\right]=\frac{NUR}{x}$$

Carbon dioxide uptake rate (CUR / mmol $L^{-1}$ $h^{-1}$), molecular hydrogen uptake rate (HUR / mmol $L^{-1}$ $h^{-1}$), $CH_4$ evolution rate (MER / mmol $L^{-1}$ $h^{-1}$), carbon balance (C-balance), yields ($Y_{(CH4/CO2)}$ and $Y_{(x/CO2)}$) and biomass productivity ($r_x$ / c-mmol $L^{-1}$ $h^{-1}$) was calculated as described elsewhere (Taubner et al. 2016; Rittmann et al. 2012, Bernacchi et al. 2014). The concentrations of $H_2$, $CO_2$, $N_2$ and $CH_4$ after GC measurements were obtained.

Results

Prioritization of strains

Growth of *M. marburgensis, M. maripaludis S0001, M. thermaggregans, M. villosus* and *M. okinawensis* was analyzed in a $H_2/CO_2/N_2$ atmosphere in defined, but $NH_4^+$ containing medium, and a N-free medium. This allowed us to screen for $NH_4^+$ uptake, $N_2$ fixation, amino acid excretion and the conversion of $H_2/CO_2$ to $CH_4$ in parallel. All methanogens but *M. thermaggregans* could be grown to an $OD_{578}$ of 0.7 in $NH_4^+$ containing medium. Further experiments showed that a certain amount of $NH_4^+$ was necessary for growth under these conditions. Due to most favorable growth characteristics in these experiments, *M. marburgensis* was selected for further experiments. Growth at specific $NH_4^+$ concentrations with and without bicarbonate ($Na_2CO_3$) was already performed and showed no or nitrogen-limited growth in 0% and 1 % and similar growth in all other concentrations. In experiments with $Na_2CO_3$ in the media, a higher growth rate was shown.

$NH_4^+$ uptake kinetics of *M. marburgensis*

Closed batch experiments of *M. marburgensis,* were then performed with 0%, 5 %, 7.5%, 10 % and 100% $NH_4^+$ in relation to original media composition of 2.1 g $L^{-1}$, gassed with $H_2/CO_2/N_2$ in the ratio 7:1:1. To reduce the possibility of a $NH_4^+$ carryover the experiments were performed with one washing step in octuplicates (n = 8) with additional zero controls. Due to the biomass washing step a slower growth compared to non-washed biomass experiments was observed. After 77.17 h the $OD_{578}$ located between 0.17 and 0.20. The 100% 4:1 positive control as expected showed the best $OD_{578}$ value around 0.25. Gas samples were taken after approximately 40, 59 and 77 h (Figure 1).
Additional fed-batch experiments (n = 3) at $NH_4^+$ concentrations of 0%, 1%, 5%, 10% and 100% were performed. Reference runs showed similar growth up to an $OD_{578}$ of 7.0 and 8.1 (Figure 1). Comparing the runs at 100% $NH_4^+$, a 2.2-fold higher $OD_{578}$ was obtained with $Na_2CO_3$ in the media. This effect was also visible in closed batch experiments. $NH_4^+$ concentration of 10% showed a stagnation after 20 h reaching a final $OD_{578}$ of 1.6, 5% displays stagnation after 15 h reaching an $OD_{578}$ of 0.9.
A detailed description of $H_2$ and $CO_2$ uptake rates, HUR and CUR, respectively, and MER is shown in Table 2.

**Table 2:** Hydrogen Uptake Rate (HUR), Carbon dioxide Uptake Rate (CUR), Nitrogen Uptake Rate (NUR) and Methane Evolution Rate (MER) and C-Balance of closed batch experiments of *M. marburgensis* with different $NH_4^+$ concentrations.

| | HUR [mmol/L*h] | CUR [mmol/L*h] | NUR [mmol/L*h] | MER [mmol/L*h] | $\Delta x$ [g/L] | $\Delta t$ | rx | Y ($CH_4/CO_2$) | Y ($x/CO_2$) | C Balance |
|---|---|---|---|---|---|---|---|---|---|---|
| 5%_39.5h | -3.19 | -0.55 | 0.03 | 1.25 | 0.02 | 20.00 | 0.00 | 2.27 | 0.00 | 2.27 |
| 5%_58.5h | -3.48 | -1.02 | 0.21 | 1.50 | 0.04 | 19.00 | 0.00 | 1.48 | 0.00 | 1.48 |
| 5%_77.17h | -2.66 | -0.96 | -0.46 | 1.45 | 0.06 | 18.67 | 0.00 | 1.51 | 0.00 | 1.51 |
| 7.5%_39.5h | -1.53 | -0.54 | -0.91 | 1.14 | 0.04 | 20.00 | 0.00 | 2.12 | 0.00 | 2.12 |
| 7.5%_58.5h | 1.41 | -0.26 | 0.24 | 1.08 | 0.05 | 19.00 | 0.00 | 4.10 | 0.00 | 4.10 |
| 7.5%_77.17h | -2.58 | -0.59 | -0.47 | 1.21 | 0.07 | 19.00 | 0.00 | 2.07 | 0.00 | 2.07 |
| 10%_39.5h | -2.63 | -0.88 | -0.30 | 0.94 | 0.04 | 20.00 | 0.00 | 1.06 | 0.00 | 1.06 |
| 10%_58.5h | -3.37 | -0.79 | -0.88 | 1.32 | 0.05 | 19.00 | 0.00 | 1.68 | 0.00 | 1.68 |
| 10%_77.17h | -4.10 | -0.60 | -0.49 | 1.26 | 0.06 | 18.75 | 0.00 | 2.11 | 0.00 | 2.11 |
| 100%_39.5h | -3.29 | -0.45 | -0.83 | 0.91 | 0.03 | 20.00 | 0.00 | 2.03 | 0.00 | 2.03 |
| 100%_58.5h | -3.73 | -0.73 | -0.54 | 0.97 | 0.05 | 19.25 | 0.00 | 1.33 | 0.00 | 1.33 |
| 100%_77.17h | -3.32 | -0.84 | -0.55 | 1.13 | 0.06 | 18.50 | 0.00 | 1.34 | 0.00 | 1.34 |

The interplay of simultaneous uptake of $NH_4^+$ and $N_2$

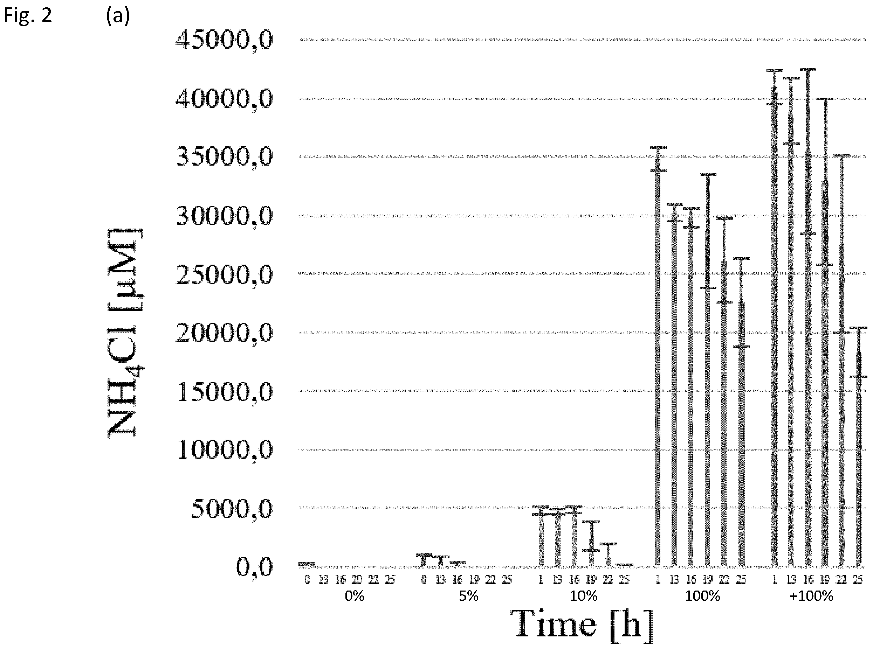

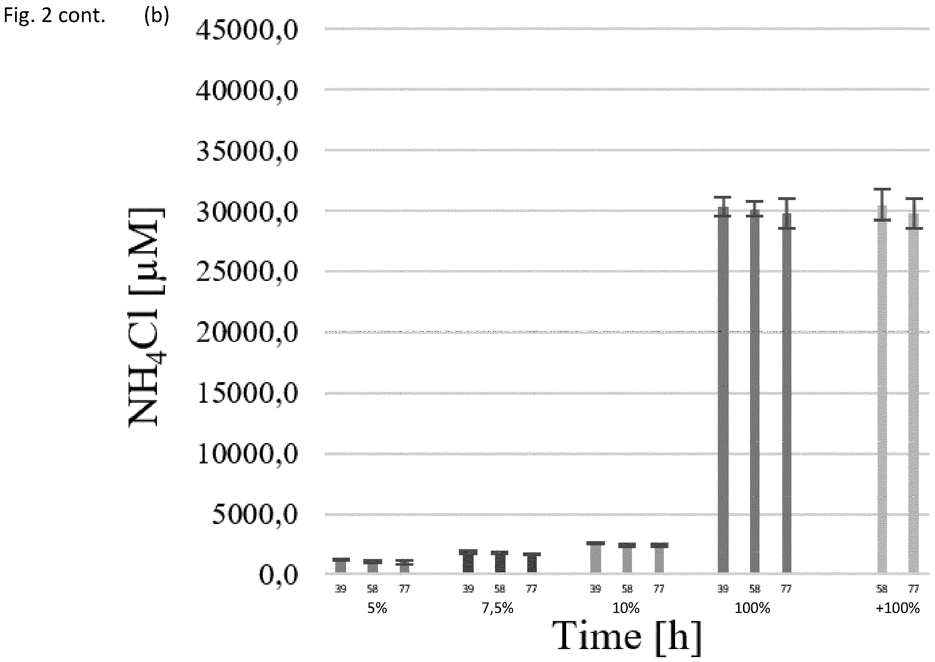

An $NH_4^+$ uptake during $N_2$ fixation is evident in closed batch and fed-batch experiments. Comparing Figure 2 (chart a) to Figure 2 (chart b) it is visible that during in fed-batch cultivation an $NH_4^+$ limitation was observed at 0-10% of $NH_4^+$, whereas in closed batch cultivation $NH_4^+$ was never completely consumed. Further, it is noticeable that within the closed batch experiments the $NH_4^+$ concentration does not differ much between time points, in contrast to fed-batch experiments where $NH_4^+$ decreases over time (Figure 2). The highest consumption was achieved in the positive control experiments. In case of closed batch experiments the highest ammonia uptake rate (AUR) was achieved within the 100% 4:1 run with 243.8 $\mu$mol L$^{-1}$ h$^{-1}$ at a $qN_2$ of 4.6 $\mu$mol h$^{-1}$ g$^{-1}$, and in case of fed-batch experiments the highest AUR of 577.3 $\mu$mol L$^{-1}$ h$^{-1}$ and $qN_2$ of 10.8 $\mu$mol h$^{-1}$ g$^{-1}$ in 100% was obtained with bicarbonate in the media.

Highest NUR was calculated from 7.5% and 100% closed batch experiments after 40 h with 0.91 or 0.83 mmol L$^{-1}$ h$^{-1}$ respectively and from 10% after 59 h with 0.88 mmol L$^{-1}$ h$^{-1}$. The $qN_2$ shows the same pattern (Table 3). Earlier sampling time points showed higher NUR, later time points lower, but more balanced NUR values.

**Table 3:** NUR [mmol L$^{-1}$ h$^{-1}$] and $qN_2$ [mmol h$^{-1}$ g$^{-1}$] of closed batch experiments in 5%, 7.5%, 10% and 100% NH$_4$Cl.

| | NUR [mmol L$^{-1}$ h$^{-1}$] | NUR SD [mmol L$^{-1}$ h$^{-1}$] | $qN_2$ [mmol h$^{-1}$ g$^{-1}$] | $qN_2$ SD [mmol h$^{-1}$ g$^{-1}$] | AUR [$\mu$mol L$^{-1}$ h$^{-1}$] | AURSD [$\mu$mol L$^{-1}$ h$^{-1}$] |
|---|---|---|---|---|---|---|
| 0%_ 77h | 0.83 | 0.10 | 298.31 | 164.27 | -1 | 0 |
| 5%_ 40h | 0.25 | 0.09 | 12.50 | 7.52 | 73 | **31** |
| 5%_ 59h | 0.23 | 0.13 | 5.52 | 3.96 | 54 | **48** |
| 5%_ 77h | 0.46 | 0.24 | 7.47 | 3.81 | 97 | 52 |
| 7.5%_ 40h | 0.91 | 0.44 | 27.08 | 13.73 | 13 | 41 |
| 7.5%_ 59h | 0.27 | 0.11 | 5.53 | 2.57 | 87 | 17 |
| 7.5%_ 77h | 0.47 | 0.08 | 7.19 | 1.40 | 92 | 12 |
| 10%_ 40h | 0.30 | 0.10 | 10.74 | 3.25 | 122 | 16 |
| 10%_ 59h | 0.88 | 0.12 | 17.05 | 2.47 | 117 | 42 |
| 10%_ 77h | 0.49 | 0.19 | 7.65 | 2.95 | 92 | 44 |
| 100%_ 40h | 0.83 | 0.13 | 29.94 | 5.91 | 109 | 285 |
| 100%_ 59h | 0.54 | 0.14 | 11.84 | 2.97 | 177 | 229 |
| 100%_ 77h | 0.55 | 0.19 | 9.30 | 3.30 | 128 | 453 |

Amino acid excretion by *M. marburgensis*

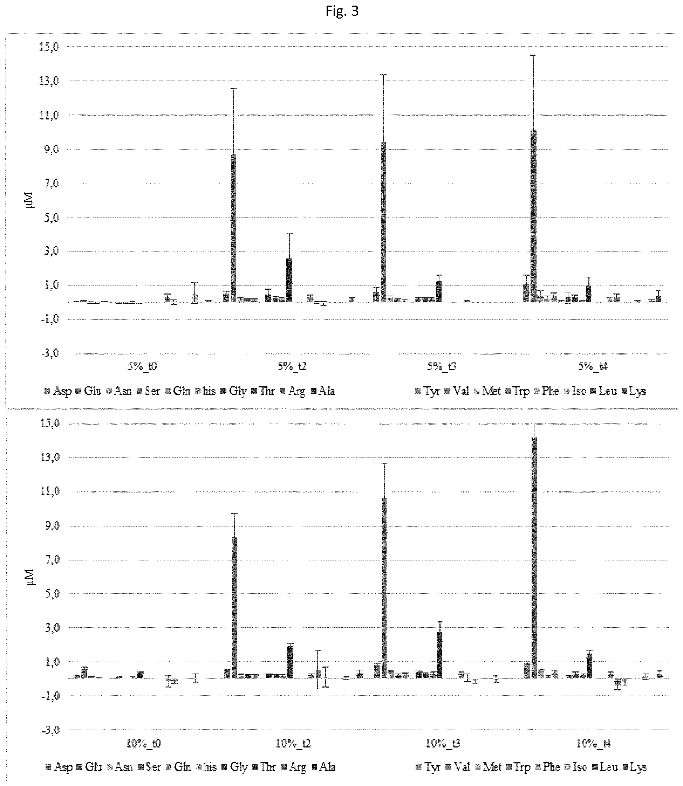

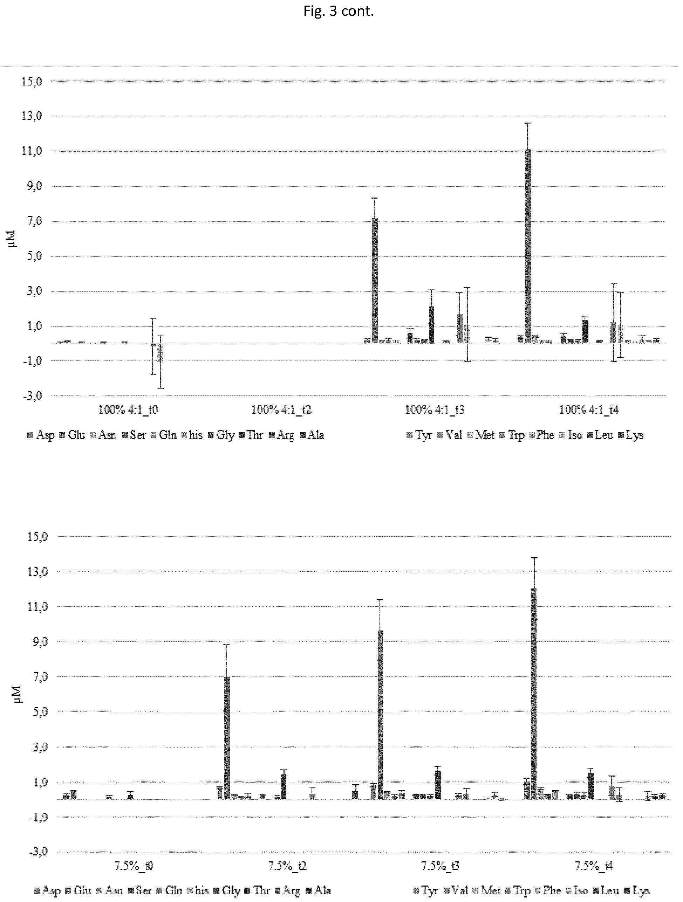

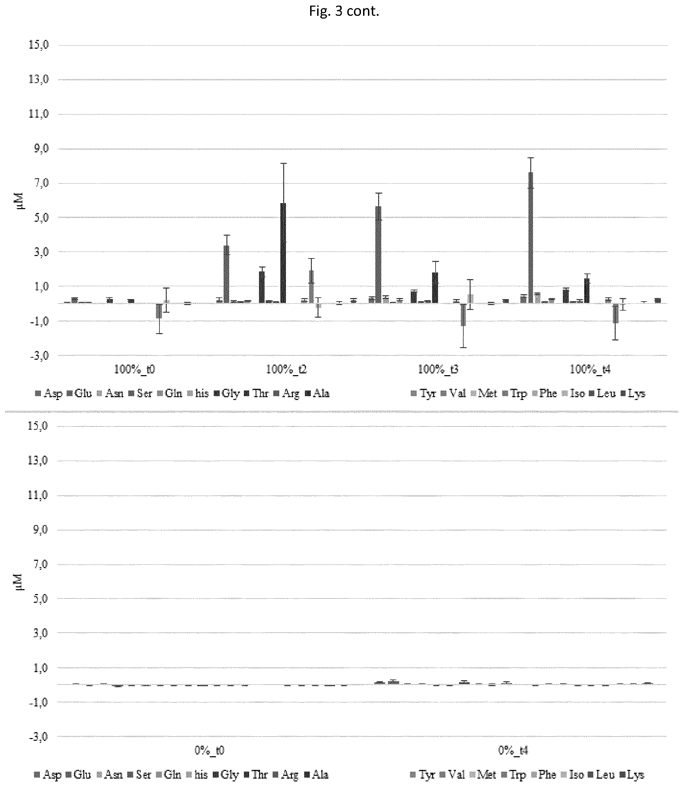

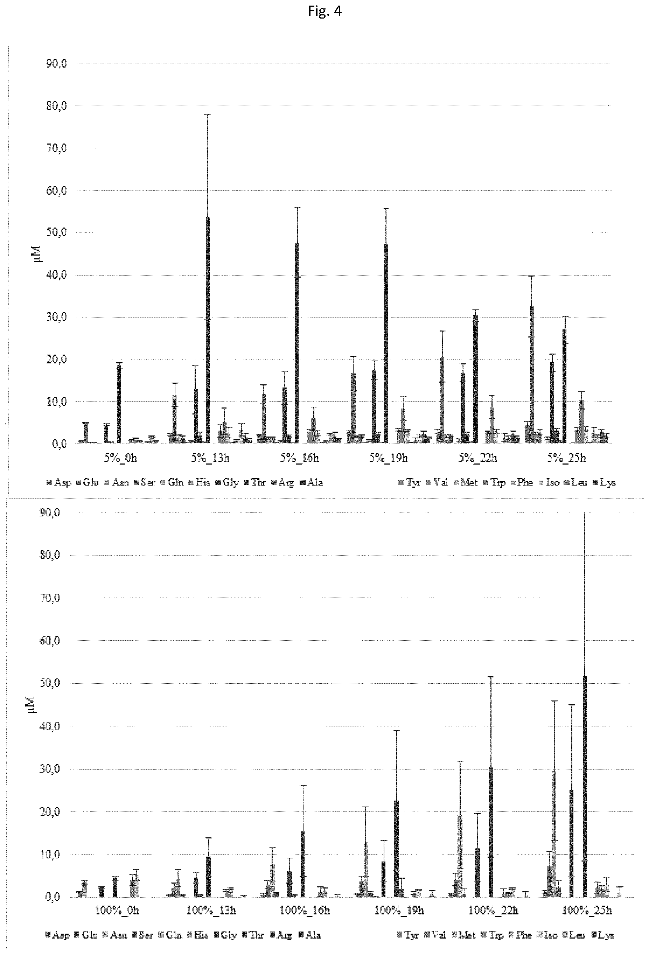

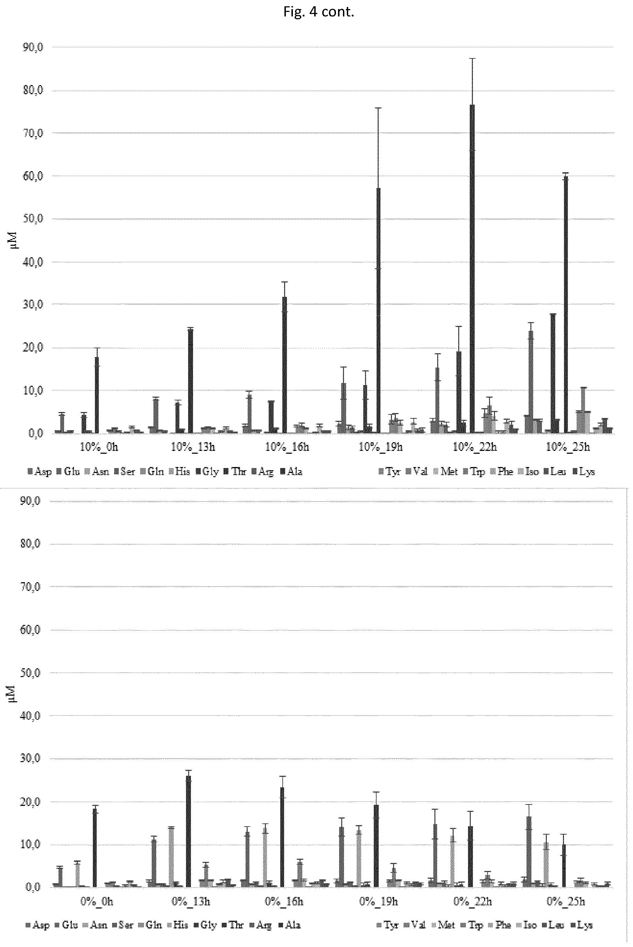

Active amino acid excretion by *M. marburgenis* was investigated in closed batch (Figure 3) and fed-batch (Figure 4) experiments. Independent of the cultivation mode almost all detectable amino acids were found. The highest excreted amino acids were glutamic acid (Glu), alanine (Ala), glycine (Gly) and asparagin (Asn). The concentrations of Glu, Gly and Asn were constantly increasing during the course of the cultivation, yet Ala was consumed after a certain timepoint. All cultivation experiments showed a clear $NH_4^+$ dependence where amino acid excretion in 5%, 7.5% and 10% varies from 100%. (Figure 3 and 4). Taking a closer look to volumetric values, in closed batch the highest value was obtained with glutamic acid with up to 4.59 $\mu$mol L$^{-1}$ h$^{-1}$ in 5% after 40 h and a smaller amout of alanine with up to

1.36 $\mu$mol L$^{-1}$ h$^{-1}$. Highest value of Gly was achieved in 100% after 40 h with 0.99 $\mu$mol L$^{-1}$ h$^{-1}$. Fed-batch cultivation lead to Ala as the most excreted amino acid, highest in 5% with up to 2.67 $\mu$mol L$^{-1}$ h$^{-1}$. It was also noticable that in 100% Asn was produced 10-fold higher in comparison with the other $NH_4^+$ concentrations with up to 0.79 $\mu$mol L$^{-1}$ h$^{-1}$.

Examining the total amount of excreted amino acids, closed batch shows an increasing excretion of amino acids over time with the highest in later timepoints with 14.67 to 18.44 $\mu$mol L$^{-1}$. Fed-batch shows in general a higher total amount of AA excretion, with highest value of 156.08 $\mu$mol L$^{-1}$ (Figure 5). On the contrary it is to note, that during closed batch experiments showed a higher production rate [$\mu$mol L$^{-1}$h$^{-1}$] than fed-batch experiments. The presence of too much $NH_4^+$ in the media seems to serve as an inhibitor for amino acid production, as 100% experiments showed slightly lower values (Figure 5). A comparison of the total uptake of $NH_4^+$ with the total amino acids excretion rate indicated that amino acid excretion rate increased with increasing AUR. Furthermore, during depletion of $NH_4^+$, the concentration of AA did not increase (Figure 5).

Conclusions

In the context of "power to gas" technology, biological methanation with $CO_2$ originating from renewable sources and in combination with $N_2$ fixation, the production of amino acids by methanogens is of high economic interest. To our knowledge there is no study yet that examined a combined $CO_2/N_2$ fixation bioprocess.

A switch of cultivation mode, closed or fed-batch, could alter amino acid excretion rates and concentration, e.g. in the case of Glu from the highest fed-batch value of 2.74 mg L$^{-1}$ to 44.83 mg L$^{-1}$ in closed batch (Table 3).

Surprisingly, a variety of amino acids turned out to be actively excreted, with the highest total amount of up to 7.5 $\mu$mol L$^{-1}$ h$^{-1}$ in early time points (Figure 5) .

These results underscore that a methanogenic microorganism is well suited for production of amino acids in a biotechnological context, even under $N_2$-fixing conditions.

**Example 2: Amino acid production and active secretion by *Methanothermobacter marburgensis* in continuous culture**

Continuous culture of *M. marburgensis* for amino acid production was successfully established.

Experiments were carried out with *M. marburgensis* in 2 L bioreactors (Eppendorf AG, Hamburg, Germany) and in a 15 L bioreactor (Biostat C+ ,Sartorius Stedim Biotech AG, Göttingen, Germany).

For fermentation, original MM medium as described in Example 1 above was used.

The same medium was used as feed medium for continuous cultivation mode.

To ensure anaerobic conditions inside the reaction vessel, the whole system was flushed with an $H_2/CO_2$, $N_2$ or $H_2/CO_2/N_2$ mixture for 10 minutes prior to inoculation.

Cultivation was performed at 65°C and a stirrer speed of 100 to 1200 rpm (DASGIP parallel bioreactor system, Eppendorf AG, Hamburg, Germany) and from 100 to 1500 rpm (Biostat C+, Sartorius Stedim Biotech AG, Göttingen, Germany). The pH was measured by a pH probe (Mettler Toledo GmbH, Vienna, Austria or Hamilton Bonaduz AG, Bonaduz, Switzerland) and kept constant at a value of 7.

The oxidation reduction potential (ORP) was measured by a redox probe (Mettler Toledo GmbH, Vienna, Austria). A 0.5 mol/L $Na_2S \cdot 9H_2O$ solution was used as sulphur source and fed constantly to the bioreactor at e.g. 0.2 mL/h to 1.32 mL/h. The MM medium was applied using an analog peristaltic pump. The MM medium feed flow rate, the sodium $Na_2S \cdot 9H_2O$ feed rate and the titration was recorded gravimetrically or adjusted by pump speed. The bioreactor volume was kept constant by withdrawing culture suspension over an immersion pipe using a peristaltic pump controlled on a fixed bioreactor weight, or by using a pipe at a fixed height as a level control system. The withdrawn suspension was collected in a harvest bottle and its volume recorded gravimetrically. All solutions were made anaerobic by flushing with $N_2$, $H_2/CO_2$, or $H_2/CO_2/N_2$. To maintain anaerobic conditions, all bottles were pressurized with $N_2$. Pure $H_2/CO_2$ (4:1) was used as substrate for *M. marburgensis*. $CO_2$ gas flow was controlled via the MX4/4 unit (Eppendorf AG, Hamburg, Germany). $H_2$ gas flow was controlled via the C100L Unit (Sierra Instruments, Monterey, USA) .

About thirty different runs of continuous cultures of *M. marburgensis* were performed under anaerobic conditions. The volume of a run ranged from 1.6 L to 10.29 L. Dilution rate D was varied between runs, in particular with values for D from 0.0125 h$^{-1}$ to 0.05 h$^{-1}$. Volume gas per volume liquid per minute (vvm) was also varied between runs, e.g. from 0.125 to 0.5. Agitation (rpm) was also varied between runs, for instance from 375 to 1500. As a sulfur source, 0.5 mol/L $Na_2S$ was supplied from e.g. 0.2 mL/h to 1.32 mL/h. Typically, the ammonium concentration was kept between 15 mmol/L and 35 mmol/L.

Importantly, volumetric amino acid production rate and specific amino acid production rate ranged from about 25 to about 75 $\mu$mol L$^{-1}$h$^{-1}$ and from about 50 to about 2000 $\mu$mol h$^{-1}$g$^{-1}$, respectively (total over all amino acids). The production and secretion into the culture supernatant of the following amino acids (in combination) was typically observed: Asp, Glu, Asn, Ser, His, Gln, Gly, Thr, Arg, Ala, Tyr, Val, Met, norvaline (Nva), Trp, Ile, Phe, Leu, Lys. Individual amino acid production rates were observed up to about 40 $\mu$mol L$^{-1}$h$^{-1}$ (volumetric) and up to about 900 $\mu$mol h$^{-1}$g$^{-1}$ (specific per biomass). Cys and Pro were not detected due to analytic contraints, but are exptected to be produced and secreted as well.

In conclusion, reliable production of amino acids was observed in continuous culture. The secretion of these amino acids to the supernatant is especially remarkable, as it simplifies downstream steps (e.g. no cell lysis required for harvesting the product). Also surprising was the observed production of Nva, which has not been observed before in methanogenic archaea (let alone in Methanobacteriales).

**Example 3: Amino acid production and active secretion in further methanogenic archaea**

Production of amino acids, including canonical amino acids and Nva, and their active secretion were observed in methanogenic archaea other than *M. marburgensis,* namely in *Methanocaldococcus jannaschii, Methanococcus igneus* and *Methanocaldococcus villosus.* These methanogenic microorganisms were incubated under closed batch conditions similar to the conditions disclosed in Example 1, but at their respective preferred temperatures in 282-based medium (see also Mauerhofer et al, 2021). While for instance Glu production was more pronounced under these conditions, Nva production was also clearly observed for each of *Methanocaldococcus jannaschii, Methanococcus igneus* and *Methanocaldococcus villosus* (volumetric Nva production rates reached beyond 1.0 $\mu$mol L$^{-1}$h$^{-1}$, specific Nva production rates beyond 10 $\mu$mol g$^{-1}$h$^{-1}$). Production of Nva has not been observed before in methanogenic archaea (let alone in Methanococcales) .

In summary, the production and active secretion into the culture supernatant of the following amino acids (in combination) was observed for methanogenic microorganisms: Asp, Glu, Asn, Ser, His, Gln, Gly, Thr, Arg, Ala, Tyr, Val, Met, Nva, Trp, Ile, Phe, Leu and Lys.

Non-patent references

Abdel Azim, A., Rittmann, S.K.-M.R., Fino, D., Bochmann, G., 2018. The physiological effect of heavy metals and volatile fatty acids on Methanococcus maripaludis S2. Biotechnol Biofuels 11, 301.

Abdel Azim, Annalisa, Christian Pruckner, Philipp Kolar, Ruth Sophie Taubner, Debora Fino, Guido Saracco, Filipa L. Sousa, and Simon K. M. R. Rittmann. 2017. "The Physiology of Trace Elements in Biological Methane Production." Bioresource Technology 241:775-86.

Becker J and Wittmann C. Systems and synthetic metabolic engineering for amino acid production - the heartbeat of industrial strain development. Curr Opin Biotechnol. 2012 Oct;23(5):718-26.

Belay N, Sparling R and Daniels L. 1984. "Dinitrogen Fixation by a Thermophilic Methanogenic Bacterium." Nature 312(5991):286-288.

Bellack, Annett, Harald Huber, Reinhard Rachel, Gerhard Wanner, and Reinhard Wirth. 2011. "Methanocaldococcus Villosus Sp. Nov., a Heavily Flagellated Archaeon That Adheres to Surfaces and Forms Cell-Cell Contacts."

Bernacchi, Sébastien, et al. "Experimental methods for screening parameters influencing the growth to product yield (Y (x/CH4)) of a biological methane production (BMP) process performed with Methanothermobacter marburgensis." AIMS Bioengineering 1.2 (2014): 72-87.

Biermann, Michael, et al. "Simultaneous analysis of the non-canonical amino acids norleucine and norvaline in biopharmaceutical-related fermentation processes by a new ultrahigh performance liquid chromatography approach." Amino Acids 44.4 (2013): 1225-1231.

Blank, C. E., Peter S. Kessler, and John A. Leigh. 1995. "Genetics in Methanogens: Transposon Insertion Mutagenesis of a Methanococcus Maripaludis NifH Gene." Journal of Bacteriology 177(20):5773-77.

Blotevogel, Karl Heinz and Ulrich Fischer. 1985. "Isolation and Characterization of a New Thermophilic and Autotrophic Methane Producing Bacterium:Methanobacterium Thermoaggregans Spec. Nov." Archives of Microbiology 142(3):218-22.

Bult, Carol J., et al. "Complete genome sequence of the methanogenic archaeon, Methanococcus jannaschii." Science 273.5278 (1996): 1058-1073.

Dhamad, Ahmed E., and Daniel J. Lessner. "A CRISPRi-dCas9 system for archaea and its use to examine gene function during nitrogen fixation by Methanosarcina acetivorans." Applied and environmental microbiology 86.21 (2020): e01402-20.

Fardeau, M-L., J-P. Peillex, and J-P. Belaich. "Energetics of the growth of Methanobacterium thermoautotrophicum and Methanococcus thermolithotrophicus on ammonium chloride and dinitrogen." Archives of microbiology 148.2 (1987): 128-131.

Fernandez L, Bertilsson S, Peura S 2019 Non-cyanobacterial diazotrophs dominate nitrogen-fixing communities in permafrost thaw ponds, Limnol. Oceanogr. 65, 2020, S180-S193, Association for the Sciences of Limnology and Oceanography.

Hu Y and Ribbe MW. 2012. "Nitrogenase Assembly." Biochimica et Biophysica Acta 1827:1112-22.

Jones, W. Jack, M. J. B. Paynter, and R. Gupta. 1983. Characterization of Methanococcus Maripaludis Sp. Nov., a New Methanogen Isolated from Salt Marsh Sediment. Vol. 135.

Kandeler, Ellen. 1988. "Short-Term Assay of Soil Urease Activity Using Colorimetric Determination of Ammonium SoilCare View Project Biodiversity Exploratories View Project." Biology and Fertility of Soils 6(1):68-72.

Kessler, Peter S., Jennifer McLarnan, and John A. Leigh. "Nitrogenase phylogeny and the molybdenum dependence of nitrogen fixation in Methanococcus maripaludis." Journal of bacteriology 179.2 (1997): 541-543.

Kurth JM, Op den Camp HJM, Welte CU, 2020. Several ways one goal-methanogenesis from unconventional substrates. Appl Microbiol Biotechnol 104, 6839-6854.

Liu, Hui, et al. "Effects of different amino acids and their configurations on methane yield and biotransformation of intermediate metabolites during anaerobic digestion." Journal of Environmental Management 296 (2021): 113152.

Leigh, John A. 2000. "Nitrogen Fixation In Methanogens: The Archaeal Perspective." 2:125-31.

Lyu, Z, Shao N, Akinyemi T, Whitman WB, 2018. Methanogenesis. Current Biology 28, R727-R732.

Mand, Thomas D., and William W. Metcalf. "Energy conservation and hydrogenase function in methanogenic archaea, in particular the genus Methanosarcina." Microbiology and Molecular Biology Reviews 83.4 (2019): e00020-19.

Mauerhofer, LM., Zwirtmayr, S., Pappenreiter, P. et al. Hyperthermophilic methanogenic archaea act as high-pressure CH4 cell factories. Commun Biol 4, 289 (2021).

Mauerhofer, L.-M., Reischl, B., Schmider, T., Schupp, B., Nagy, K., Pappenreiter, P., Zwirtmayr, S., Schuster, B., Bernacchi, S., Seifert, A.H., Paulik, C., Rittmann, S.K.-M.R., 2018. Physiology and methane productivity of Methanobacterium thermaggregans. Appl Microbiol Biotechnol 102, 7643-7656.

Mayumi D, Hanako M, Hideyuki T, Kyosuke Y, Hideyoshi Y, Yuichiro S, Yoichi K, and Susumu S. 2016. "Methane Production from Coal by a Single Methanogen." Science 354(6309):222-25.

Murray PA and Zinder SH. 1984. "Nitrogen Fixation by a Methanogenic Archaebacterium." Nature 312(5991):284-86.

Nayak, Dipti D., and William W. Metcalf. "Cas9-mediated genome editing in the methanogenic archaeon Methanosarcina acetivorans." Proceedings of the National Academy of Sciences 114.11 (2017): 2976-2981.

Pappenreiter, P.A., Zwirtmayr, S., Mauerhofer, L.-M., Rittmann, S.K.-M.R., Paulik, C., 2019. Development of a simultaneous bioreactor system for characterization of gas production kinetics of methanogenic archaea at high pressure. Engineering in Life Sciences 19, 537-544.

Pfeifer, Kevin, et al. "Archaea biotechnology." Biotechnology Advances 47 (2021): 107668.

Polis, Baruh, et al. "L-Norvaline, a new therapeutic agent against Alzheimer's disease." Neural regeneration research 14.9 (2019): 1562.

Raymond J, Siefert JL, Staples CR, and Blankenship RE. 2004. "The Natural History of Nitrogen Fixation." Molecular Biology and Evolution 21(3):541-54.

Rittmann, S., A. Seifert, and Christoph Herwig. "Quantitative analysis of media dilution rate effects on Methanothermobacter marburgensis grown in continuous culture on H2 and CO2." Biomass and Bioenergy 36 (2012): 293-301.

Rittmann, S.K.-M.R., 2015. A Critical Assessment of Microbiological Biogas to Biomethane Upgrading Systems. Adv. Biochem. Eng. Biotechnol. 151, 117-135.

Rittmann, S.K.-M.R., Seifert, A.H., Bernacchi, S., 2018. Kinetics, multivariate statistical modelling, and physiology of CO2-based biological methane production. Applied Energy 216, 751-760.

Rittmann, Simon K-MR, et al. "Archaea in der Biotechnologie." BIOspektrum 27.1 (2021): 96-98.

Sarmiento, Felipe B., John A. Leigh, and William B. Whitman. "Genetic systems for hydrogenotrophic methanogens." Methods in enzymology. Vol. 494. Academic Press, 2011. 43-73.

Schönheit P, Johanna Moll, and Rudolf K. Thauer. 1980. "Growth Parameters (Ks, Mmax, Ys) of Methanobacterium Thermoautotrophicum." Archives of Microbiology 127(1):59-65

Takai, Ken, Akira Inoue, and Koki Horikoshi. 2002. "Methanothermococcus Okinawensis Sp. Nov., a Thermophilic, Methane-Producing Archaeon Isolated from a Western Pacific Deep-Sea Hydrothermal Vent System." International Journal of Systematic and Evolutionary Microbiology 52(4):1089-95.

Taubner, Ruth Sophie and Simon K. M. R. Rittmann. 2016. "Method for Indirect Quantification of CH4production via H2O Production Using Hydrogenotrophic Methanogens." Frontiers in Microbiology 7(APR):1-11.

Taubner, R.-S., et al, 2018. Biological methane production under putative Enceladus-like conditions. Nature Communications 9, 748.

Taubner, Ruth-Sophie, et al. "Membrane lipid composition and amino acid excretion patterns of Methanothermococcus okinawensis grown in the presence of inhibitors detected in the Enceladian plume." Life 9.4 (2019): 85.

Thamdrup B ,New Pathways and Processes in the Global Nitrogen Cycle, 2012, Annual Review of Ecology, Evolution, and Systematics, 407-428, 43, 1.

Wasserfallen A, et al. 2000. "Phylogenetic Analysis of 18 Thermophilic Methanobacterium Isolates Supports the Proposals to Create a New Genus, Methanothermobacter Gen. Nov., and to Reclassify Several Isolates in Three Species, Methanothermobacter Thermautotrophicus Comb. Nov., Methano." International Journal of Systematic and Evolutionary Microbiology 50:43-53.

## Claims

1. A method of producing amino acids by fermentation in a bioreactor, wherein the bioreactor comprises methanogenic microorganisms in a fermentation broth, the method comprising at least the step of feeding a gaseous carbon source comprising carbon dioxide and/or carbon monoxide, a nitrogen source comprising nitrogen gas and preferably a sulfur source to the bioreactor under conditions such that the methanogenic microorganisms produce the amino acids, wherein the fermentation broth comprises ammonium at a concentration of at least 0.1 mmol/L and the tungstate concentration in the fermentation broth is below 0.1 $\mu$mol/L, preferably below 0.01 $\mu$mol/L, especially below 0.001 $\mu$mol/L.

2. The method of claim 1, wherein the fermentation broth is essentially free of tungstate.

3. The method of claim 1 or 2, wherein hydrogen gas, acetate, a methyl compound preferably selected from methylamines, methyl sulfides and methanol, any other alcohol, preferably a secondary alcohol such as 2-propanol or 2-butanol, a methoxylated aromatic compound and/or formate are fed to the bioreactor; preferably wherein hydrogen gas, acetate, methanol or combinations thereof are fed to the bioreactor.

4. The method of any one of claims 1 to 3, further comprising the step of harvesting at least a portion of the amino acids from the bioreactor.

5. The method of any one of claims 1 to 4, wherein the amino acids further comprise, or wherein said portion further comprises, at least one, preferably at least two or even at least three, more preferably at least four or even at least five, even more preferably at least seven or even at least nine, yet more preferably at least 12 or even at least 15, yet even more preferably at least 17 or even at least 18, especially all of the 20 canonical amino acids.

6. The method of any one of claims 1 to 5, wherein the methanogenic microorganisms comprise archaea selected from any of Methanobacteriales, Methanococcales, Methanomicrobiales, Methanosarcinales, Methanopyrales, Methanocellales, Methanomassiliicoccales, and Methanonatronarchaeales, preferably Methanobacteriales and Methanococcales; more preferably selected from Methanobacteriaceae, Methanocaldococcaceae and Methanococcaceae; in particular selected from *Methanothermobacter, Methanothermococcus, Methanocaldococcus* and *Methanococcus.*

7. The method of any one of claims 1 to 6, wherein the total amino acid production rate per volume of fermentation broth is at least 0.01 $\mu$mol L$^{-1}$h$^{-1}$, preferably at least 0.05 $\mu$mol L$^{-1}$h$^{-1}$, more preferably at least 0.1 $\mu$mol L$^{-1}$h$^{-1}$, even more preferably at least 0.5 $\mu$mol L$^{-1}$h$^{-1}$, yet even more preferably at least 1.0 $\mu$mol L$^{-1}$h$^{-1}$, especially at least 5 $\mu$mol L$^{-1}$h$^{-1}$ or even at least 10 $\mu$mol L$^{-1}$h$^{-1}$.

8. The method of any one of claims 1 to 7, wherein the total amino acid production rate per biomass is at least 0.1 $\mu$mol g$^{-1}$h$^{-1}$, preferably at least 0.5 $\mu$mol g$^{-1}$h$^{-1}$, more preferably at least 1.0 $\mu$mol g$^{-1}$h$^{-1}$, even more preferably at least 5 $\mu$mol g$^{-1}$h$^{-1}$, yet even more preferably at least 10 $\mu$mol g$^{-1}$h$^{-1}$, especially at least 50 $\mu$mol g$^{-1}$h$^{-1}$ or even at least 100 $\mu$mol g$^{-1}$h$^{-1}$.

9. The method of any one of claims 1 to 8, wherein the method is a continuous process, a fed-batch process, a batch process, a closed batch process, a repetitive batch process, a repetitive fed-batch process or a repetitive closed batch process, preferably a continuous process, a fed-batch process or a repetitive fed-batch process, especially a continuous process.

10. The method of any one of claims 1 to 9, wherein at least 50%, preferably at least 60%, more preferably at least 70%, even more preferably at least 80%, yet even more preferably at least 90% or even more preferably at least 95%, especially at least 99% or even at least 99.9% of all nitrogen atoms of all nitrogen sources fed to the bioreactor are fed to the bioreactor in the form of nitrogen gas.

11. The method of any one of claims 1 to 10, wherein at least 50%, preferably at least 60%, more preferably at least 70%, even more preferably at least 80%, yet even more preferably at least 90% or even more preferably at least 95%, especially at least 99% or even at least 99.9% of all carbon atoms of all carbon sources fed to the bioreactor are fed to the bioreactor in the form of carbon dioxide gas and/or carbon monoxide gas.

12. The method of any one of claims 1 to 11, wherein the fermentation is started with the methanogenic microorganisms in chemically defined fermentation medium.

13. Use of methanogenic microorganisms for producing amino acids from an electron donor compound, a gaseous carbon source comprising carbon dioxide and/or carbon monoxide, a nitrogen source comprising nitrogen gas and preferably a sulfur source, wherein the fermentation broth comprises ammonium at a concentration of at least 0.1 mmol/L and the tungstate concentration in the fermentation broth is below 0.1 $\mu$mol/L, preferably below 0.01 $\mu$mol/L, especially below 0.001 $\mu$mol/L.

14. A fermentation broth comprising methanogenic microorganisms, wherein the fermentation broth comprises ammonium at a concentration of at least 0.1 mmol/L and the tungstate concentration in the fermentation broth is below

0.1 $\mu$mol/L, preferably below 0.01 $\mu$mol/L, especially below 0.001 $\mu$mol/L.

15. A bioreactor comprising the fermentation broth of claim 14.

(a)

Fig. 1

EP 4 296 354 A1

Fig. 1 cont.

EP 4 296 354 A1

EP 4 296 354 A1

Fig. 2      (a)

Fig. 2 cont.    (b)

EP 4 296 354 A1

Fig. 3

Fig. 3 cont.

Fig. 3 cont.

Fig. 4

Fig. 4 cont.

Fig. 5

(a)                                                          (b)

| | Europäisches Patentamt European Patent Office Office européen des brevets | **EUROPEAN SEARCH REPORT** | **Application Number** EP 22 18 0986 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | M-L FARDEAU ET AL: "Energetics of the growth of Methanobacterium thermoautotrophicum and Methanococcus thermolithotrophicus on ammonium chloride and dinitrogen", ARCHIVES OF MICROBIOLOGY, SPRINGER BERLIN HEIDELBERG, BERLIN/HEIDELBERG, vol. 148, no. 2, 1 February 1987 (1987-02-01), pages 128-131, XP009532718, ISSN: 0302-8933, DOI: 10.1007/BF00425360 | 14,15 | INV. C12N1/20 C12P13/04 |
| Y | * page 148, right-hand column, paragraph 3 - page 149, left-hand column, paragraph 3 * | 1-13 | |
| | ----- | | |
| X | Whitman William B ET AL: "Nutrition and Carbon Metabolism of Methanococcus voltae", JOURNAL OF BACTERIOLOGY., 1 January 1982 (1982-01-01), pages 852-863, XP055982279, Retrieved from the Internet: URL:https://www.ncbi.nlm.nih.gov/pmc/articles/PMC216471/ [retrieved on 2022-11-16] | 14,15 | |
| | | | **TECHNICAL FIELDS SEARCHED (IPC)** C12N C12P |
| Y | * abstract * * page 852, right-hand column, last paragraph - page 853, left-hand column, paragraph 3 * | 1-13 | |
| | ----- | | |
| | -/-- | | |

The present search report has been drawn up for all claims

| Place of search | Date of completion of the search | Examiner |
|---|---|---|
| Berlin | 22 November 2022 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 22 18 0986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SMENT K A ET AL: "Excretion of amino acids by 1,2,4-triazole-3-alanine-resistant mutants of Methanococcus voltae", APPLIED AND ENVIRONMENTAL MICROBIOLOGY, vol. 55, no. 5, 1 May 1989 (1989-05-01), pages 1295-1297, XP055982287, US ISSN: 0099-2240, DOI: 10.1128/aem.55.5.1295-1297.1989 * abstract * * page 1295, left-hand column, paragraph 1 – right-hand column, paragraph 4; tables 1,2 * | 1-13 | |
| Y | WO 2017/070726 A1 (TECHNISCHE UNIVERSITÄT WIEN [AT]) 4 May 2017 (2017-05-04) * page 1, lines 3-4 * * page 2, line 9 – page 5, line 2 * * page 12, line 12 – page 13, line 8; examples 1-5 * | 1-13 | |
| Y | HOFFARTH MARC PHILIPPE ET AL: "Effect of N2 on Biological Methanation in a Continuous Stirred-Tank Reactor with Methanothermobacter marburgensis", FERMENTATION, vol. 5, no. 3, 2 July 2019 (2019-07-02), page 56, XP055982791, DOI: 10.3390/fermentation5030056 * page 2, paragraph 8-11 * * page 12, line 24 – page 14, line 26 * | 1-13 | TECHNICAL FIELDS SEARCHED (IPC) |

-/--

The present search report has been drawn up for all claims

| Place of search | Date of completion of the search | Examiner |
|---|---|---|
| Berlin | 22 November 2022 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EUROPEAN SEARCH REPORT

Application Number

EP 22 18 0986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2016/179545 A1 (TRELYS INC [US]) 10 November 2016 (2016-11-10) * page 16, line 3 - page 52, line 24 * * page 53, line 4 - page 56, line 28 * * page 60, line 28 - page 63, line 16 * * examples 1,9 * | 1-13 | |
| Y | WHITMAN WILLIAM B ET AL: "Isolation and characterization of 22 mesophilic methanococci", SYSTEMATIC AND APPLIED MICROBIOLOGY, URBAN & FISCHER, AMSTERDAM, NL , vol. 7, no. 2-3 1 January 1986 (1986-01-01), pages 235-240, XP009540663, ISSN: 0723-2020, DOI: 10.1016/S0723-2020(86)80012-1 Retrieved from the Internet: URL:https://api.elsevier.com/content/artic le/PII:S0723202086800121?httpAccept=text/p lain * page 236, left-hand column, last paragraph - right-hand column, paragraph 5; table 1 * | 1-13 | TECHNICAL FIELDS SEARCHED (IPC) |
| Y | WO 2020/252335 A1 (TRELYS INC [US]) 17 December 2020 (2020-12-17) * pages 1-2, paragraph [0004] * * page 10, paragraph [0036] - page 15, paragraph [0055] * * page 17, paragraph [0065] - page 18, paragraph [0068] * * page 29, paragraph [00104] * * examples 1,5,9-11 * | 1-13 | |

-/--

The present search report has been drawn up for all claims

| Place of search | Date of completion of the search | Examiner |
|---|---|---|
| Berlin | 22 November 2022 | Mateo Rosell, A |

EPO FORM 1503 03.82 (P04C01)

## EUROPEAN SEARCH REPORT

Application Number

EP 22 18 0986

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JONES ET AL: "Methanococcus vannielii: culture and effects of selenium and tungsten on growth.", JOURNAL OF BACTERIOLOGY, vol. 130, no. 3, 1 June 1977 (1977-06-01), pages 1404-1406, XP055021192, ISSN: 0021-9193 * page 1440, left-hand column, paragraph 1 * * page 1404, right-hand column, last paragraph – page 1406, left-hand column, paragraph 1; figure 1 * | 1-15 | |
| Y | BÉDIS DRIDI ET AL: "Tungsten-enhanced growth of Methanosphaera stadtmanae", BMC RESEARCH NOTES, BIOMED CENTRAL LTD, GB, vol. 5, no. 1, 15 May 2012 (2012-05-15), page 238, XP021117144, ISSN: 1756-0500, DOI: 10.1186/1756-0500-5-238 * abstract * * page 2, right-hand column, paragraph 1 – page 3, right-hand column, paragraph 3; figures 1,2; tables 1,2 * | 1-15 | |

TECHNICAL FIELDS
SEARCHED (IPC)

The present search report has been drawn up for all claims

| Place of search | Date of completion of the search | Examiner |
|---|---|---|
| Berlin | 22 November 2022 | Mateo Rosell, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

page 4 of 4

## ANNEX TO THE EUROPEAN SEARCH REPORT
## ON EUROPEAN PATENT APPLICATION NO.

EP 22 18 0986

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2022

| Patent document cited in search report | | Publication date | Patent family member(s) | | Publication date |
|---|---|---|---|---|---|
| WO 2017070726 | A1 | 04-05-2017 | AT | 517476 A4 | 15-02-2017 |
| | | | WO 2017070726 A1 | | 04-05-2017 |
| WO 2016179545 | A1 | 10-11-2016 | AU | 2016258183 A1 | 16-11-2017 |
| | | | BR | 112017023761 A2 | 31-07-2018 |
| | | | CA | 2984797 A1 | 10-11-2016 |
| | | | CN | 107743520 A | 27-02-2018 |
| | | | EP | 3292208 A1 | 14-03-2018 |
| | | | JP | 2018516552 A | 28-06-2018 |
| | | | KR | 20180002700 A | 08-01-2018 |
| | | | RU | 2017142360 A | 06-06-2019 |
| | | | SG | 11201708741Y A | 29-11-2017 |
| | | | US | 2018163240 A1 | 14-06-2018 |
| | | | US | 2020095623 A1 | 26-03-2020 |
| | | | WO | 2016179545 A1 | 10-11-2016 |
| WO 2020252335 | A1 | 17-12-2020 | NONE | | |

EPO FORM P0459

For more details about this annex : see Official Journal of the European Patent Office, No. 12/82

## REFERENCES CITED IN THE DESCRIPTION

### Patent documents cited in the description

- WO 2012110256 A1
- WO 2014128300 A1
- WO 2017070726 A1
- WO 2016179545 A1
- US 20190194630 A1
- WO 2020252335 A1
- US 11260039 B1
- CN 106520651 A
- US 20060057685 A1

### Non-patent literature cited in the description

- **ABDEL AZIM, A. ; RITTMANN, S.K.-M.R. ; FINO, D. ; BOCHMANN, G.** The physiological effect of heavy metals and volatile fatty acids on Methanococcus maripaludis S2. *Biotechnol Biofuels,* 2018, vol. 11, 301
- **ABDEL AZIM ; ANNALISA ; CHRISTIAN PRUCKNER ; PHILIPP KOLAR ; UTH SOPHIE TAUBNER ; DEBORA FINO ; GUIDO SARACCO ; FILIPA L. SOUSA ; SIMON K. M. R. RITTMANN.** The Physiology of Trace Elements in Biological Methane Production. *Bioresource Technology,* 2017, vol. 241, 775-86
- **BECKER J ; WITTMANN C.** Systems and synthetic metabolic engineering for amino acid production - the heartbeat of industrial strain development. *Curr Opin Biotechnol.,* October 2012, vol. 23 (5), 718-26
- **BELAY N ; SPARLING R ; DANIELS L.** Dinitrogen Fixation by a Thermophilic Methanogenic Bacterium. *Nature,* 1984, vol. 312 (5991), 286-288
- **BELLACK ; ANNETT ; HARALD HUBER ; REINHARD RACHEL ; GERHARD WANNER ; REINHARD WIRTH.** *Methanocaldococcus Villosus Sp. Nov., a Heavily Flagellated Archaeon That Adheres to Surfaces and Forms Cell-Cell Contacts.,* 2011
- **BERNACCHI, SÉBASTIEN et al.** Experimental methods for screening parameters influencing the growth to product yield (Y (x/CH4)) of a biological methane production (BMP) process performed with Methanothermobacter marburgensis. *AIMS Bioengineering,* 2014, vol. 1 (2), 72-87
- **BIERMANN, MICHAEL et al.** Simultaneous analysis of the non-canonical amino acids norleucine and norvaline in biopharmaceutical-related fermentation processes by a new ultrahigh performance liquid chromatography approach. *Amino Acids,* 2013, vol. 44 (4), 1225-1231
- **BLANK, C. E. ; PETER S. KESSLER ; JOHN A. LEIGH.** Genetics in Methanogens: Transposon Insertion Mutagenesis of a Methanococcus Maripaludis NifH Gene. *Journal of Bacteriology,* 1995, vol. 177 (20), 5773-77
- **BLOTEVOGEL ; KARL HEINZ ; ULRICH FISCHER.** Isolation and Characterization of a New Thermophilic and Autotrophic Methane Producing Bacterium:Methanobacterium Thermoaggregans Spec. Nov. *Archives of Microbiology,* 1985, vol. 142 (3), 218-22
- **BULT ; CAROL J. et al.** Complete genome sequence of the methanogenic archaeon, Methanococcus jannaschii. *Science,* 1996, vol. 273 (5278), 1058-1073
- **DHAMAD ; AHMED E. ; DANIEL J. LESSNER.** A CRISPRi-dCas9 system for archaea and its use to examine gene function during nitrogen fixation by Methanosarcina acetivorans. *Applied and environmental microbiology,* 2020, vol. 86 (21), e01402-20
- **FARDEAU, M-L. ; J-P. PEILLEX ; J-P. BELAICH.** Energetics of the growth of Methanobacterium thermoautotrophicum and Methanococcus thermolithotrophicus on ammonium chloride and dinitrogen. *Archives of microbiology,* 1987, vol. 148 (2), 128-131
- **FERNANDEZ L ; BERTILSSON S ; PEURA S.** Non-cyanobacterial diazotrophs dominate nitrogen-fixing communities in permafrost thaw ponds. *Limnol. Oceanogr.,* 2019, vol. 65, S180-S193
- **HU Y ; RIBBE MW.** Nitrogenase Assembly. *Biochimica et Biophysica Acta,* 2012, vol. 1827, 1112-22
- **JONES, W. ; JACK, M. ; J. B. PAYNTER ; R. GUPTA.** Characterization of Methanococcus Maripaludis Sp. Nov. *a New Methanogen Isolated from Salt Marsh Sediment,* 1983, vol. 135

- **KANDELER ; ELLEN.** Short-Term Assay of Soil Urease Activity Using Colorimetric Determination of Ammonium SoilCare View Project Biodiversity Exploratories View Project. *Biology and Fertility of Soils,* 1988, vol. 6 (1), 68-72
- **KESSLER, PETER S. ; JENNIFER MCLARNAN ; JOHN A. LEIGH.** Nitrogenase phylogeny and the molybdenum dependence of nitrogen fixation in Methanococcus maripaludis. *Journal of bacteriology,* 1997, vol. 179 (2), 541-543
- **KURTH JM ; OP DEN CAMP HJM ; WELTE CU.** Several ways one goal-methanogenesis from unconventional substrates. *Appl Microbiol Biotechnol,* 2020, vol. 104, 6839-6854
- **LIU, HUI et al.** Effects of different amino acids and their configurations on methane yield and biotransformation of intermediate metabolites during anaerobic digestion. *Journal of Environmental Management,* 2021, vol. 296, 113152
- **LEIGH, JOHN A.** *Nitrogen Fixation In Methanogens: The Archaeal Perspective.,* 2000, vol. 2, 125-31
- **LYU, Z ; SHAO N ; AKINYEMI T ; WHITMAN WB.** *Methanogenesis. Current Biology,* 2018, vol. 28, R727-R732
- **MAND, THOMAS D. ; WILLIAM W. METCALF.** Energy conservation and hydrogenase function in methanogenic archaea, in particular the genus Methanosarcina. *Microbiology and Molecular Biology Reviews,* 2019, vol. 83 (4), e00020-19
- **MAUERHOFER, LM. ; ZWIRTMAYR, S. ; PAPPENREITER, P et al.** Hyperthermophilic methanogenic archaea act as high-pressure CH4 cell factories. *Commun Biol,* 2021, vol. 4, 289
- **MAUERHOFER, L.-M. ; REISCHL, B. ; SCHMIDER, T. ; SCHUPP, B. ; NAGY, K. ; PAPPENREITER, P. ; ZWIRTMAYR, S. ; SCHUSTER, B. ; BERNACCHI, S. ; SEIFERT, A.H.** Physiology and methane productivity of Methanobacterium thermaggregans. *Appl Microbiol Biotechnol,* 2018, vol. 102, 7643-7656
- **MAYUMI D ; HANAKO M ; HIDEYUKI T ; KYOSUKE Y ; HIDEYOSHI Y ; YUICHIRO S ; YOICHI K ; SUSUMU S.** Methane Production from Coal by a Single Methanogen. *Science,* 2016, vol. 354 (6309), 222-25
- **MURRAY PA ; ZINDER SH.** Nitrogen Fixation by a Methanogenic Archaebacterium. *Nature,* 1984, vol. 312 (5991), 284-86
- **NAYAK, DIPTI D. ; WILLIAM W. METCALF.** Cas9-mediated genome editing in the methanogenic archaeon Methanosarcina acetivorans. *Proceedings of the National Academy of Sciences,* 2017, vol. 114 (11), 2976-2981
- **PAPPENREITER, P.A. ; ZWIRTMAYR, S. ; MAUERHOFER, L.-M. ; RITTMANN, S.K.-M.R. ; PAULIK, C.** Development of a simultaneous bioreactor system for characterization of gas production kinetics of methanogenic archaea at high pressure. *Engineering in Life Sciences,* 2019, vol. 19, 537-544
- **PFEIFER, KEVIN et al.** Archaea biotechnology. *Biotechnology Advances,* 2021, vol. 47, 107668
- **POLIS, BARUH et al.** L-Norvaline, a new therapeutic agent against Alzheimer's disease. *Neural regeneration research,* 2019, vol. 14 (9), 1562
- **RAYMOND J ; SIEFERT JL ; STAPLES CR ; BLANKENSHIP RE.** The Natural History of Nitrogen Fixation. *Molecular Biology and Evolution,* 2004, vol. 21 (3), 541-54
- **RITTMANN, S. ; A. SEIFERT ; CHRISTOPH HERWIG.** Quantitative analysis of media dilution rate effects on Methanothermobacter marburgensis grown in continuous culture on H2 and CO2. *Biomass and Bioenergy,* 2012, vol. 36, 293-301
- **RITTMANN, S.K.-M.R.** A Critical Assessment of Microbiological Biogas to Biomethane Upgrading Systems. *Adv. Biochem. Eng. Biotechnol.,* 2015, vol. 151, 117-135
- **RITTMANN, S.K.-M.R. ; SEIFERT, A.H. ; BERNACCHI, S.** Kinetics, multivariate statistical modelling, and physiology of CO2-based biological methane production. *Applied Energy,* 2018, vol. 216, 751-760
- **RITTMANN ; SIMON K-MR et al.** Archaea in der Biotechnologie. *BIOspektrum,* 2021, vol. 27 (1), 96-98
- Genetic systems for hydrogenotrophic methanogens. **SARMIENTO ; FELIPE B. ; JOHN A. LEIGH ; WILLIAM B. WHITMAN.** Methods in enzymology. Academic Press, 2011, vol. 494, 43-73
- **SCHÖNHEIT P ; JOHANNA MOLL ; RUDOLF K. THAUER.** Growth Parameters (Ks, Mmax, Ys) of Methanobacterium Thermoautotrophicum. *Archives of Microbiology,* 1980, vol. 127 (1), 59-65
- **TAKAI ; KEN ; AKIRA INOUE ; KOKI HORIKOSHI.** Methanothermococcus Okinawensis Sp. Nov., a Thermophilic, Methane-Producing Archaeon Isolated from a Western Pacific Deep-Sea Hydrothermal Vent System. *International Journal of Systematic and Evolutionary Microbiology,* 2002, vol. 52 (4), 1089-95
- **TAUBNER ; RUTH SOPHIE ; SIMON K. M. R. RITTMANN.** Method for Indirect Quantification of CH4production via H2O Production Using Hydrogenotrophic Methanogens. *Frontiers in Microbiology,* 2016, vol. 7, 1-11
- **TAUBNER, R.-S. et al.** Biological methane production under putative Enceladus-like conditions. *Nature Communications,* 2018, vol. 9, 748

- **TAUBNER ; RUTH-SOPHIE et al.** Membrane lipid composition and amino acid excretion patterns of Methanothermococcus okinawensis grown in the presence of inhibitors detected in the Enceladian plume. *Life,* 2019, vol. 9 (4), 85
- **THAMDRUP B.** New Pathways and Processes in the Global Nitrogen Cycle. *Annual Review of Ecology, Evolution, and Systematics,* 2012, vol. 407-428 (43), 1

- **WASSERFALLEN A et al.** Phylogenetic Analysis of 18 Thermophilic Methanobacterium Isolates Supports the Proposals to Create a New Genus, Methanothermobacter Gen. Nov., and to Reclassify Several Isolates in Three Species, Methanothermobacter Thermautotrophicus Comb. Nov., Methano. *International Journal of Systematic and Evolutionary Microbiology,* 2000, vol. 50, 43-53